# EUROPEAN PATENT APPLICATION

(11) **EP 4 092 029 A1**
(43) Date of publication of application: **23.11.2022**
(21) Application number: 20913360.2
(22) Date of filing: 14.07.2020
(51) Int. Cl.: C07D 487/04, A61K 31/519, A61P 35/00

(54) **7-AMINO-3,4-DIHYDROPYRIMIDOPYRIMIDIN-2-ONE DERIVATIVE HAVING INHIBITORY ACTIVITY FOR PROTEIN KINASES AND THERAPEUTIC PHARMACEUTICAL COMPOSITION COMPRISING SAME**

(30) Priority: 15.01.2020 KR 20200005305
(71) Applicant: Korea Institute of Science and Technology, Seoul 02792 (KR)
(72) Inventor: SIM, Tae Bo, Seoul 02792 (KR); CHO, Han Na, Seoul 02792 (KR); CHOI, Seung Hye, Seoul 02792 (KR); HUR, Woo Young, Seoul 02792 (KR); SONG, Chi Man, Seoul 02792 (KR); LEE, Ji Hoon, Seoul 01626 (KR); LEE, Jung Yeol, Daegu 41122 (KR); PARK, Sun You, Daegu 42782 (KR); CHOI, Myeong A, Daegu 42782 (KR); JOO, Jeong Min, Daegu 41072 (KR)
(74) Representative: Weiss, Wolfgang
(86) International application number: PCT/KR2020/009258
(87) International publication number: WO 2021/145520

(57) **Abstract**

The present disclosure relates to a 7-amino-3,4-dihydropyrimidopyrimidin-2-one derivative compound exhibiting excellent anti-proliferative effects against cancer cells, a pharmaceutically acceptable salt thereof, a hydrate thereof, or a stereoisomer thereof, a production method therefor, a pharmaceutical composition for preventing, alleviating or treating cancer metastasis and proliferative disease containing the same as an active ingredient, and an anti-cancer composition against cancer cells. The compound exhibits excellent cancer cell inhibitory activity and anti-proliferative effects, and thus is effective in inhibiting cancer cells, preventing cancer metastasis and proliferative diseases or treating cancer.

## Description

### Technical Field

The present disclosure relates to a compound selected from among a novel 7-amino-3,4-dihydropyrimidopyrimidin-2-one derivative having protein kinase inhibitory activity, a pharmaceutically acceptable salt thereof, a hydrate thereof, and a stereoisomer thereof, a method for producing the compound, and a pharmaceutical composition for preventing, alleviating or treating cancer containing the compound.

### Background Art

Protein kinases are enzymes that catalyze phosphorylation reactions that transfer a gamma-phosphate group from ATP to the hydroxyl groups of tyrosine, serine and threonine on a protein. They are involved in cellular metabolism, gene expression, cell growth, differentiation and cell division, and play an important role in cell signaling.

Protein kinases account for about 2% of the eukaryotic genome, and there are about 518 protein kinases in the human genome. Protein kinases are classified into tyrosine protein kinases that phosphorylate tyrosine, and serine/threonine kinases that phosphorylate serine and threonine. Among them, more than about 90 kinases are tyrosine kinases, and are divided into receptor tyrosine kinases (RTKs) and non-receptor tyrosine kinases (NRTKs). Receptor tyrosine kinases are membrane proteins that have domains capable of receiving growth factors on the cell surface, and have active sites capable of phosphorylating tyrosine residues in the cytoplasm. Non-receptor tyrosine kinases are single tyrosine kinase domains present in the nucleus and cytoplasm, and phosphorylate tyrosine residues by receiving signals even though they are not receptors.

Protein kinases are molecular switches and the transition between active and inactive states thereof in cells should be smoothly regulated. When they are abnormally regulated, they excessively activate excessive intracellular signaling, resulting in uncontrollable cell division and proliferation. In addition, abnormal activation of protein kinases by gene mutation, amplification and overexpression is associated with the development and progression of various tumors, and thus plays a decisive role in the growth and metastasis of cancer cells. Typical examples of protein kinases that are abnormally regulated include EGFR, VEGFR, PDGFRB, c-KIT, ABL1, SRC, BRAF, FGFR, BTK, SYK, ALK, MET, CDK, MEK, mTOR, JAK, LCK, PLK, RSK, LYN, FMS, TIE2, RET, AKT, MAP, FAK, DDR, FLT3, and FES. In particular, since receptor tyrosine kinases is mainly involved in external signaling pathways for cell growth and signaling pathways for internal responses, inhibition thereof may also lead to cancer cell growth inhibition and death.

Due to these characteristics, inhibition of kinase activity has attracted attention as a major target for the development of anticancer drugs, and studies on the development of low-molecular-weight organic compounds targeting various kinases have been actively conducted.

Examples of kinase inhibitors include Gleevec^{®} (imatinib, Novartis) which is a Bcr-Abl and PDGFR tyrosine kinase inhibitor, Herceptin^{®} (trastuzumab, Genentech) which is an Her-2 antibody, Iressa^{®} (gefitinib, AstraZeneca) which is an EGFR inhibitor, Nexavar^{®} (sorafenib, Bayer) which is an inhibitor of Raf, VEGFR, KIT, RET, PDGFR-B and FLT-3, Zelboraf^{®} (vemurafenib, Roche) which is a BRAF inhibitor, Erbitux^{®} (cetuximab, Imclone) which is an EGFR antibody, Tarceva^{®} (erlotinib, Genentech/Roche) which is an EGFR inhibitor, and Sutent^{®} (sunitinib, Pfizer) which is a KDR inhibitor. They have been approved by the FDA for use as anticancer drugs for leukemia, breast cancer, non-small cell lung cancer, liver cancer, malignant melanoma, colorectal cancer and the like, and are widely used as first-line standard therapy due to their excellent therapeutic efficacy. In addition, various compounds are in clinical trials.

Meanwhile, acute myeloid leukemia (AML) is one of fatal blood diseases in which blood cells differentiate abnormally and proliferate continuously. More than 16% of acute myeloid leukemia (AML) patients have a point-mutated RAS (small G protein) protein, and NRAS mutations account for the majority (10% or more) of RAS kinases. For this reason, NRAS G protein has been considered as a promising drug target for the treatment of AML. When the protooncogene RAS is mutated, RAS is continuously activated (gain-of-function), and various signaling pathways downstream of RAS are activated to accelerate cancer cell growth.

Over the last 40 years, RAS point mutations or key signaling molecules downstream of RAS have been proposed as targets. However, they did not lead to *in vivo* experiments and clinical trials due to the complexity and compensatory effect of mutant RAS signaling pathways. For example, selumetinib (AZD 6244), which inhibits MEK, a key molecule downstream of RAS, showed no therapeutic effect in all three AML patients with a NRAS mutant gene in phase 2 clinical trials. In addition, in an attempt to find targets, RNA interference screening was used to identify proteins (TBK1, STK33 and GATA2) which are genetically in a synthetic lethal relationship with KRAS mutation. However, this also failed to achieve clinical therapeutic effects. In particular, in the case of STK33, it was proven through cell-based pharmacological screening at the preclinical stage that therapeutic strategies using KRAS mutation and synthetic lethal principles cannot be established. In addition, in recent years, cell-based pharmacological screening was used to identify a compound (GNF 7) that selectively inhibits the RAS mutant signaling pathway, and the inhibitory effects thereof in a preclinical leukemia model were confirmed. The mechanism of action of the GNF 7 compound is to simultaneously inhibit two kinases, GCK and ACK1, which specifically contribute to cell growth downstream of the RAS mutant signaling pathway. This compound actually exhibited its efficacy even in cell samples from AML patients with NRAS mutation. Inhibitors against two kinases, GCK and ACK1, are known to be effective for the treatment of cancer diseases caused by NRAS mutation, such as melanoma, colorectal cancer, thyroid cancer, and various blood cancers.

### [Prior Art Documents]

### [Patent Documents]

(Patent Document 1) International Patent Publication No. WO 2015-011597

### [Non-Patent Documents]

(Non-Patent Document 1) Choi HG, Ren P, Adrian F, et al. A type-II kinase inhibitor capable of inhibiting the T315I "gatekeeper" mutant of Bcr-Abl. J. Med. Chem., 2010; 53(15): 5439-5448.
(Non-Patent Document 2) Nonami, A.; Sattler, M.; Weisberg, E.; Liu, Q.; Zhang, J.; Patricelli, M. P.; Christie, A. L.; Saur, A. M.; Kohl, N. E.; Kung, A. L.; Yoon, H.; Sim, T.; Gray, N. S.; Griffin, J. D., Identification of novel therapeutic targets in acute leukemias with NRAS mutations using a pharmacologic approach. Blood 2015, 125 (20), 3133-43.
(Non-Patent Document 3) Luo T, Masson K, Jaffe JD, et al. STK33 kinase inhibitor BRD-8899 has no effect on KRASdependent cancer cell viability. Proc Natl Acad Sci USA. 2012; 109(8): 2860-2865.
(Non-Patent Document 4) Jain N, Curran E, Iyengar NM, et al. Phase II study of the oral MEK inhibitor selumetinib in advanced acute myelogenous leukemia:a University of Chicago phase II consortium trial. Clin Cancer Res. 2014; 20(2): 490-498.
(Non-Patent Document 5) Johnson, D. B.; Smalley, K. S.; Sosman, J. A., Molecular pathways: targeting NRAS in melanoma and acute myelogenous leukemia. Clin Cancer Res 2014, 20(16), 4186-92.
(Non-Patent Document 6) H Cho, I Shin, E Ju, et al. First SAR Study for Overriding NRAS Mutant Driven Acute Myeloid Leukemia. J. Med. Chem. 2018, 61 (18), 8353-8373.

### DISCLOSURE

### Technical Problem

Therefore, an object of the present disclosure is to provide a novel 7-amino-3,4-dihydropyrimidopyrimidin-2-one derivative compound having protein kinase inhibitory activity.

Another object of the present disclosure is to provide a pharmaceutical composition useful for the treatment, prevention and alleviation of cancer disease, the pharmaceutical composition containing, as an active ingredient, a novel 7-amino-3,4-dihydropyrimidopyrimidin-2-one derivative compound, a pharmaceutically acceptable salt thereof, a hydrate thereof, a solvate thereof, or a stereoisomer thereof.

Still another object of the present disclosure is to provide a therapeutic agent for cancer disease caused by NRAS mutation, the therapeutic agent containing, as an active ingredient, a novel 7-amino-3,4-dihydropyrimidopyrimidin-2-one derivative compound, a pharmaceutically acceptable salt thereof, a hydrate thereof, a solvate thereof, or a stereoisomer thereof.

### Technical Solution

To achieve the above objects, the present disclosure provides a compound selected from among a 7-amino-3,4-dihydropyrimidopyrimidin-2-one derivative compound represented by the following Formula 1, a pharmaceutically acceptable salt thereof, a hydrate thereof, and a stereoisomer thereof:

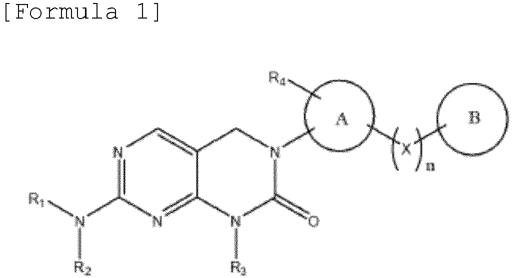

wherein
R₁ is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl), or R₁ together with a nitrogen atom to which R₂ is attached forms a 4- to 7-membered saturated, unsaturated or aromatic ring, which may optionally contain at least one of N, O, S, NH, C=N, C=O, -NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ is hydrogen, a C₁-C₁₃ alkyl group, a C₃-C₁₀ cyclyl group, or a C₃-C₁₀ heterocyclyl group, or R₂ together with a nitrogen atom to which R₁ is attached forms a 4- to 7-membered saturated, unsaturated or aromatic ring, which may optionally contain at least one of N, O, S, NH, C=N, C=O, - NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₃ is hydrogen, a C₁-C₁₃ alkyl group, a C₃-C₁₀ cyclyl group, or a C₃-C₁₀ heterocyclyl group;
R₄ is hydrogen, a hydroxyl group, a halogen group, a C₁-C₁₃ alkyl group, a C₁-C₆ alkoxy group, a C₁-C₆ alkenyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl) ;
A is a C₆-C₁₀ aryl group, a C₃-C₁₀ cyclyl group, a C₃-C₁₀ heteroaryl group, or a C₃-C₁₀ heterocyclyl group;
n is an integer ranging from 0 to 3;
when n is 1 or more, X is each independently selected from the group consisting of -CH₂-, -CR₅R₆-, -O-, -OC(O)-, - C(O)O-, -OS(O)₂-, -S(O)₂O-, -NR₆-, -NR₆CH₂-, -NR₆C(O)-, - C(O)NR₆-, -NR₆C(O)NR₆-, -S(O)₂-, -NR₆S(O)₂-, and -S(O)₂NR₆-;
B is a C₆-C₁₀ aryl group, a C₃-C₁₀ cyclyl group, a C₃-C₁₀ heteroaryl group, or C₃-C₁₀ heterocyclyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphoryl group (-P(O)R₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ are each independently hydrogen, a C₁-C₆ alkyl group, a C₁-C₆ alkenyl group, a C₁-C₆ alkynyl group, a C₆-C₁₀ aryl group, a C₃-C₁₀ heteroaryl group, or a C₃-C₁₀ heterocyclyl group, or R₅ together with a nitrogen or carbon atom to which R₆ is attached forms a 3- to 7-membered saturated ring, which may optionally contain at least one of N, O, S, NH, C=N, C=O, -NHC(O)-, -NHC(O)NH-, - NHS(O)₂- and SO₂ and may optionally be substituted with at least one of hydrogen, a C₁-C₁₃ alkyl group, a C₆-C₁₀ aryl group, a C₃-C₁₀ heteroaryl group, a hydroxyl group, a halide group and a cyano group; and
the C₃-C₁₀ heteroaryl group and the C₃-C₁₀ heterocyclyl group contain at least one heteroatom selected from the group consisting of N, O and S.

### Advantageous Effects

The compound according to the present disclosure has excellent ability to inhibit the activity of protein kinase such as ABL1, FGFR2, TAOK2/TAO1, EPHA5, EPHB2, EPHB3, RET, LYN B, EPHA2, FRK/PTK5, EPHA8, LCK, EPHB4, FYN, KHS/MAP4K5, DDR1, EPHA3, P38a/MAPK14, EPHA4, FMS, EPHB1, HCK, FGFR1, ABL2/ARG, EPHA6, c-Src, ACK1, FLT4/VEGFR3, ERBB4/HER4, DDR2, KDR/VEGFR2, LYN, ZAK/MLTK, YES/YES1, BLK, FGR, MLCK2/MYLK2, TAOK1, BMX/ETK, BTK, EPHA1, JAK1, P38b/MAPK11, TIE2/TEK, FLT1/VEGFR1, TXK, SRMS, RAF1, SIK1, MLK3/MAP3K11, PEAK1, TRKA, EPHA7, GLK/MAP4K3, MLK2/MAP3K10, TEC, CSK, TRKC, FES/FPS, SIK2, FGFR3, BRK, YSK4/MAP3K19, ARAF, PDGFRb, TNK1, GCK/MAP4K2, PDGFRa, TNIK, TAK1, ERBB2/HER2, LIMK1, HIPK4, FER, EGFR, JAK2, HPK1/MAP4K1, TRKB, RIPK3, LOK/STK10, LIMK2, MLK1/MAP3K9, BRAF, MEKK3, MEK5, STK32B/YANK2, FGFR4, MEKK2, SLK/STK2, FLT3, PKAcg, TAOK3/JIK, TYRO3/SKY, SIK3, IR, LRRK2, PYK2, NEK11, p70S6K/RPS6KB1, or LATS2. Thus, the compound may be used for the purpose of treating, preventing and alleviating cancer disease caused by abnormal cell growth.

The compound according to the present disclosure, a pharmaceutically acceptable salt thereof, or a hydrate thereof, and a pharmaceutical composition for preventing or treating cancer containing the same as an active ingredient exhibits high inhibitory activity and antiproliferative effects selectively against cancer cells while showing low cytotoxicity, and thus may be effectively used for the prevention or treatment of cancer.

Examples of cancer diseases that may be treated, prevented and alleviated by treatment with the compound according to the present disclosure include stomach cancer, lung cancer, liver cancer, colorectal cancer, small intestine cancer, pancreatic cancer, brain cancer, bone cancer, melanoma, breast cancer, sclerosing adenosis, uterine cancer, cervical cancer, head and neck cancer, esophageal cancer, thyroid cancer, parathyroid cancer, kidney cancer, sarcoma, prostate cancer, urethral cancer, bladder cancer, blood cancer (including leukemia, multiple myeloma, and myelodysplastic syndrome), lymphoma (Hodgkin's disease, and non-Hodgkin's lymphoma), psoriasis, or fibroadenoma.

In particular, the compound according to the present disclosure has excellent inhibitory activity against two kinases, GCK and ACK1, and thus is effective for the treatment of cancer disease caused by NRAS mutation, such as melanoma, colorectal cancer, thyroid cancer, or acute myeloid leukemia (AML).

### Best Mode

Since all numbers, values and/or expressions referring to quantities of components, reaction conditions, and mixtures used in the present specification are subject to various uncertainties of measurement encountered in obtaining such values, unless otherwise indicated, all are to be understood as modified in all instances by the term "about." Where a numerical range is disclosed herein, such a range is continuous, inclusive of both the minimum and maximum values of the range as well as every value between such minimum and maximum values, unless otherwise indicated. Still further, where such a range refers to integers, every integer between the minimum and maximum values of such a range is included, unless otherwise indicated.

In the present specification, where a range is stated for a parameter, it will be understood that the parameter includes all values within the stated range, inclusive of the stated endpoints of the range. For example, a range of 5 to 10 will be understood to include the values 5, 6, 7, 8, 9, and 10, as well as any sub-range such as 6 to 10, 7 to 10, 6 to 9, and 7 to 9, and also include any value and range between the integers which are reasonable in the context of the range stated, such as 5.5, 6.5, 7.5, 5.5 to 8.5 and 6.5 to 9. For example, a range of "10% to 30%" will be understood to include the values 10%, 11%, 12%, 13%, etc., and all integers up to and including 30%, as well as any sub-range such as 10% to 15%, 12% to 18%, 20% to 30%, etc., and also include any value between the integers which are reasonable in the context of the range stated, such as 10.5%, 15.5%, 25.5%, etc.

Hereinafter, the present disclosure will be described in detail.

The present inventors have conducted extensive studies to solve the above-described problems, and as a result, have developed an anticancer compound exhibiting excellent inhibitory activity against cancer cells, particularly a 7-amino-3,4-dihydropyrimidopyrimidin-2-one derivative compound useful for the prevention or treatment of cancer as a selective kinase activity inhibitor, a pharmaceutically acceptable salt thereof, a hydrate thereof, and a stereoisomer thereof, or a method for producing the same, and a pharmaceutical composition for preventing or treating cancer containing the same as an active ingredient.

One aspect of the present disclosure provides a compound selected from among a 7-amino-3,4-dihydropyrimidopyrimidin-2-one derivative compound represented by the following Formula 1, a pharmaceutically acceptable salt thereof, a hydrate thereof, and a stereoisomer thereof: 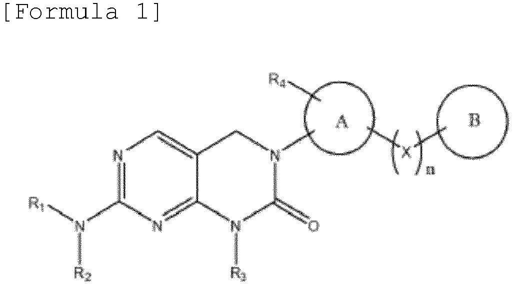 wherein
R₁ is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl), or R₁ together with a nitrogen atom to which R₂ is attached forms a 4- to 7-membered saturated, unsaturated or aromatic ring, which may optionally contain at least one of N, O, S, NH, C=N, C=O, -NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ is hydrogen, a C₁-C₁₃ alkyl group, a C₃-C₁₀ cyclyl group, or a C₃-C₁₀ heterocyclyl group, or R₂ together with a nitrogen atom to which R₁ is attached forms a 4- to 7-membered saturated, unsaturated or aromatic ring, which may optionally contain at least one of N, O, S, NH, C=N, C=O, - NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₃ is hydrogen, a C₁-C₁₃ alkyl group, a C₃-C₁₀ cyclyl group, or a C₃-C₁₀ heterocyclyl group;
R₄ is hydrogen, a hydroxyl group, a halogen group, a C₁-C₁₃ alkyl group, a C₁-C₆ alkoxy group, a C₁-C₆ alkenyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl);
A is a C₆-C₁₀ aryl group, a C₃-C₁₀ cyclyl group, a C₃-C₁₀ heteroaryl group, or a C₃-C₁₀ heterocyclyl group;
n is an integer ranging from 0 to 3;
when n is more than 1, X is each independently selected from the group consisting of -CH₂-, -CR₅R₆-, -O-, - OC(O)-, -C(O)O-, -OS(O)₂-, -S(O)₂O-, -NR₆-, -NR₆CH₂-, - NR₆C(O)-, -C(O)NR₆-, -NR₆C(O)NR₆-, -S(O)₂-, -NR₆S(O)₂-, and - S(O)₂NR₆-;
B is a C₆-C₁₀ aryl group, a C₃-C₁₀ cyclyl group, a C₃-C₁₀ heteroaryl group, or C₃-C₁₀ heterocyclyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ are each independently hydrogen, a C₁-C₆ alkyl group, a C₁-C₆ alkenyl group, a C₁-C₆ alkynyl group, a C₆-C₁₀ aryl group, a C₃-C₁₀ heteroaryl group, or a C₃-C₁₀ heterocyclyl group, or R₅ together with a nitrogen or carbon atom to which R₆ is attached forms a 3- to 7-membered saturated ring, which may optionally contain at least one of N, O, S, NH, C=N, C=O, -NHC(O)-, -NHC(O)NH-, - NHS(O)₂- and SO₂ and may optionally be substituted with at least one of hydrogen, a C₁-C₁₃ alkyl group, a C₆-C₁₀ aryl group, a C₃-C₁₀ heteroaryl group, a hydroxyl group, a halide group and a cyano group; and
the C₃-C₁₀ heteroaryl group and the C₃-C₁₀ heterocyclyl group contain at least one heteroatom selected from the group consisting of N, O and S.

In the definition of substituents in the present disclosure, the term "alkyl" refers to an aliphatic hydrocarbon radical. The alkyl may be either saturated alkyl that does not contain an alkenyl or alkynyl moiety, or unsaturated alkyl that contains at least one alkenyl or alkynyl moiety. The term "alkenyl" refers to a group containing at least one carbon-carbon double bond, and the term "alkynyl" refers to a group containing at least one carbon-carbon triple bond. The alkyl may be cyclic, branched or straight-chain when used alone or in combination.

The term "aryl" as used herein, either alone or in combination with another radical, refers to a carbocyclic aromatic monocyclic group containing 6 carbon atoms, which may be further fused to a second 5- or 6-membered carbocyclic group which may be aromatic, saturated or unsaturated. Examples of aryl include, but are not limited to, phenyl, indanyl, 1-naphthyl, 2-naphthyl, and tetrahydronaphthyl. The aryl may be connected to another group at a suitable position on the aromatic ring.

The term "alkoxy" refers to an alkyl group attached via an oxygen atom to another group (i.e., -O-alkyl). The alkoxy group may be unsubstituted or substituted with one or more suitable substituents. Examples of the alkoxy group include, but are not limited to, (C₁-C₆) alkoxy groups, for example, -O-methyl, -O-ethyl, -O-propyl, -O-isopropyl, -O-2-methyl-1-propyl, -O-2-methyl-2-propyl, -O-2-methyl-1-butyl, -O-3-methyl-1-butyl, -O-2-methyl-3-butyl, -O-2,2-dimethyl-1-propyl, -O-2-methyl-1-pentyl, 3-O-methyl-1-pentyl, -O-4-methyl-1-pentyl, -O-2-methyl-2-pentyl, -O-3-methyl-2-pentyl, -O-4-methyl-2-pentyl, -O-2,2-dimethyl-1-butyl, -O-3,3-dimethyl-butyl, -O-2-ethyl-1-butyl, -O-butyl, -O-isobutyl, -O-t-butyl, -O-pentyl, -O-isopentyl, -O-neopentyl, and -O-hexyl.

The term "phenoxy" refers to a phenyl group attached via an oxygen atom to another group (i.e., -O-aryl) . The phenoxy group may be unsubstituted or substituted with one or more halogens, alkyl groups, aryl groups or heteroaryl groups, but is not limited thereto.

The term "amino group" refers to an alkyl group attached via a nitrogen atom to another group (i.e., -NH- or -N-alkyl). The amino group may be unsubstituted or substituted with one or more suitable substituents. Examples of the amine group include, but are not limited to, (C₁-C₆) amino groups, for example, -NH-methyl, -NH-ethyl, - NH-propyl, -NH-isopropyl, -NH-2-methyl-1-propyl, -NH-2-methyl-2-propyl, -NH-2-methyl-1-butyl, -NH-3-methyl-1-butyl, -NH-2-methyl-3-butyl, -NH-2,2-dimethyl-1-propyl, -NH-2-methyl-1-pentyl, 3-NH-methyl-1-pentyl, -NH-4-methyl-1-pentyl, -NH-2-methyl-2-pentyl, -NH-3-methyl-2-pentyl, -NH-4-methyl-2-pentyl, -NH-2,2-dimethyl-1-butyl, -NH-3,3-dimethyl-butyl, -NH-2-ethyl-1-butyl, -NH-butyl, -NH-isobutyl, -NH-t-butyl, -NH-pentyl, -NH-isopentyl, -NH-neopentyl, -NH-hexyl, -N,N-dimethyl, -N-methyl-N-ethyl, -N-methyl-N-propyl, -N-methyl-isopropyl, -N-methyl-N-butyl, - N-methyl-N-isobutyl, -N-methyl-N-pentyl, -N-methyl-N-isopentyl, N-methyl-N-hexyl, N-methyl-N-isohexyl, -N,N-diethyl, -N-ethyl-N-propyl, -N-ethyl-N-isopropyl, -N-ethyl-N-butyl, -N-ethyl-N-isobutyl, -N-ethyl-N-pentyl, -N-ethyl-N-isopentyl, -N-ethyl-N-hexyl, -N-ethyl-N-isohexyl, -N,N-dipropyl, -N-propyl-N-isopropyl, -N-propyl-N-butyl, -N-propyl-N-isobutyl, -N-propyl-N-pentyl, -N-propyl-N-isopentyl, -N-propyl-N-hexyl, -N-propyl-N-isohexyl, -N,N-dibutyl, -N-butyl-N-isobutyl, -N-butyl-N-pentyl, - N-butyl-N-isopentyl, -N-butyl-N-hexyl, -N-butyl-N-isohexyl, -N,N-dipentyl, -N-pentyl-N-hexyl, -N-pentyl- N-isohexyl, and - N,N-dihexyl.

The term "halogen group" refers to fluorine, chlorine, bromine or iodine.

The term "heterocyclyl group" refers to a heteroaromatic compound containing at least one heteroatom selected from the group consisting of N, O and S, unless otherwise stated. Preferably, examples of the heterocyclyl group include, but are not limited to, a pyrrolidine group, a furan group, a morpholine group, a piperazine group and a piperidine group, more preferably a pyrrolidine group, a piperidine group, a piperazine group, and a morpholine group.

The term "heteroaryl group" refers to a heteroaromatic compound containing at least one heteroatom selected from the group consisting of N, O and S, unless otherwise stated. Preferably, examples of the heteroaryl group include, but are not limited to, a pyridine group, a pyrazine group, a pyrimidine group, a pyridazine group, a pyrazole group, an imidazole group, a triazole group, an indole group, an oxadiazole group, a thiadiazole group, a quinolone group, an isoquinoline group, an isoxazole group, an oxazole group, a thiazolyl group, and a pyrrole group.

In one aspect of the present disclosure, there is provided a compound selected from among the 7-amino-3,4-dihydropyrimidopyrimidin-2-one derivative compound represented by Formula 1, a pharmaceutically acceptable salt thereof, a hydrate thereof, and a stereoisomer thereof, wherein B is any one selected from the group consisting of a C₁-C₆ alkyl group, benzene, thiazole, thiophene, pyrazole, benzothiophene, pyridazine, pyrazine, imidazole, oxadiazole, triazole, furan, pyrimidine, oxazole, pyrrole, pyridine, oxadiazole, triazine, thiadiazole, isoxazole, and tetrazole, and B is substituted or unsubstituted.

In one aspect of the present disclosure, there is provided a compound selected from among the 7-amino-3,4-dihydropyrimidopyrimidin-2-one derivative compound represented by Formula 1, a pharmaceutically acceptable salt thereof, a hydrate thereof, and a stereoisomer thereof, wherein R₂ is hydrogen, R₃ is methyl, and R₄ is methyl.

In one aspect of the present disclosure, there is provided a compound selected from among the 7-amino-3,4-dihydropyrimidopyrimidin-2-one derivative compound represented by Formula 1, a pharmaceutically acceptable salt thereof, a hydrate thereof, and a stereoisomer thereof, wherein R₁ is any one selected from the group consisting of hydrogen, a C₁-C₆ alkyl group, a C₃-C₁₀ cyclyl group, benzene, thiazole, thiophene, pyrazole, benzothiophene, pyridazine, pyrazine, imidazole, oxadiazole, triazole, furan, pyrimidine, oxazole, pyrrole, pyridine, oxadiazole, triazine, thiadiazole, isoxazole, and tetrazole, and R₁ is substituted or unsubstituted.

In one aspect of the present disclosure, there is provided a compound selected from among the 7-amino-3,4-dihydropyrimidopyrimidin-2-one derivative compound represented by Formula 1, a pharmaceutically acceptable salt thereof, a hydrate thereof, and a stereoisomer thereof, wherein the compound is any one selected from the group consisting of the following Compound Nos. 1 to 65:
(Compound No. 1) 3-(5-(5-(2-methoxyphenyl)-1H-imidazol-2-yl)-2-methylphenyl)-1-methyl-7-((1-methyl-1H-pyrazol-4-yl)amino)-3,4-dihydropyrimido[4,5-d]pyrimidin-2(1H)-one;
(Compound No. 2) 7-((2,6-dimethylpyridin-3-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 3) 3-(5-(5-(2-methoxyphenyl)-1H-imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 4) 7-((1-(1-ethylpiperidin-4-yl)-1H-pyrazol-4-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 5) 7-((6-(1-ethylpiperidin-4-yl)pyridin-3-yl)amino)-3-(5-(5-(2-methoxyphenyl)-1H-imidazole-2-yl)-2-methylphenyl)-1-methyl-3,4-dihydropyrimido[4,5-d]pyrimidin-2(1H)-one;
(Compound No. 6) 7-((6-(4-acetylpiperazin-1-yl)pyridin-3-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 7) 7-((1-(1-acetylpiperidin-4-yl)-1H-pyrazol-4-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 8) 7-((2,6-dimethylpyridin-3-yl)amino)-1-methyl-3-(2-methyl-5-(5-(2-(trifluoromethoxy)phenyl)-1H-imidazole-2-yl)phenyl)-3,4-dihydropyrimido[4,5-d]pyrimidin-2(1H)-one;
(Compound No. 9) 1-methyl-3-(2-methyl-5-(5-(2-(trifluoromethoxy)phenyl)-1H-imidazol-2-yl)phenyl)-7-((6-methylpyridin-3-yl)amino)-3,4-dihydropyrimido[4,5-d]pyrimidin-2(1H)-one;
(Compound No. 10) 7-((1-(1-ethylpiperidin-4-yl)-1H-pyrazol-4-yl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 11) 7-(cyclopropylamine)-3-(5-(5-(2,3-difluorophenyl)-1H-imidazol-2-yl)-2-methylphenyl)-1-methyl-3,4-dihydropyrimido[4,5-d]pyrimidin-2(1H)-one;
(Compound No. 12) 3-(5-(5-(2,3-difluorophenyl)-1H-imidazol-2-yl)-2-methylphenyl)-1-methyl-7-((6-methylpyridin-3-yl))amino)-3,4-dihydropyrimido[4,5-d]pyrimidin-2(1H)-one;
(Compound No. 13) 1-methyl-3-(2-methyl-5-(5-(3-(trifluoromethyl)phenyl)-1H-imidazol-2-yl)phenyl)-7-((6-methylpyridin-3-yl)amino)-3,4-dihydropyrimido[4,5-d]pyrimidin-2(1H)-one;
(Compound No. 14) 7-((6-(4-acetylpiperazin-1-yl)pyridin-3-yl)amino)-1-methyl-3-(2-methyl-5-(5-(2-(trifloromethoxy)phenyl)-1H-imidazol-2-yl)phenyl)-3,4-dihydropyrimido[4,5-d]pyrimidin-2(1H)-one;
(Compound No. 15) 7-((6-(1-ethylpiperidin-4-yl)pyridin-3-yl)amino)-1-methyl-3-(2-methyl-5-(5-(2-(trifloromethoxy)phenyl)-1H-imidazol-2-yl)phenyl)-3,4-dihydropyrimido[4,5-d]pyrimidin-2(1H)-one;
(Compound No. 16) 7-((6-(4-ethylpiperazin-1-yl)pyridin-3-yl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 17) 7-((1-(1-acetylpiperidin-4-yl)-1H-pyrazol-4-yl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 18) 1-methyl-3-(2-methyl-5-(5-(2-(trifluoromethoxy)phenyl)-1H-imidazol-2-yl)phenyl)-7-((4-morpholinophenyl)amino)-3,4-dihydropyrimido[4,5-d]pyrimidin-2(1H)-one;
(Compound No. 19) 1-methyl-3-(2-methyl-5-(5-(2-(trifluoromethoxy)phenyl)-1H-imidazol-2-yl)phenyl)-7-(phenylamino)-3,4-dihydropyrimido[4,5-d]pyrimidin-2(1H)-one;
(Compound No. 20) 7-((4-methoxybenzyl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 21) 7-(benzylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 22) 7-(cyclopropylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 23) 7-(cyclopentylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 24) 7-(cyclohexylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 25) 7-(butylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 26) 1-methyl-3-(2-methyl-5-(5-(2-(trifluoromethoxy)phenyl)-1H-imidazol-2-yl)phenyl)-7-(methylamino)-3,4-dihydropyrimido[4,5-d]pyrimidin-2(1H)-one;
(Compound No. 27) 7-amino-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 28) 7-((6-(4-ethylpiperazin-1-yl)pyridin-3-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 29) 7-((1-(1-acetylpiperidin-4-yl)-1H-pyrazol-4-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 30) 3-(5-(5-(2-methoxyphenyl)-1H-imidazol-2-yl)-2-methylphenyl)-1-methyl-7-(phenylamino)-3,4-dihydropyrimido[4,5-d]pyrimidin-2(1H)-one;
(Compound No. 31) 7-(benzylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 32) 7-((4-methoxybenzyl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 33) 3-(5-(5-(2-methoxyphenyl)-1H-imidazol-2-yl)-2-methylphenyl)-1-methyl-7-(methylamino)-3,4-dihydropyrimido[4,5-d]pyrimidin-2(1H)-one;
(Compound No. 34) 7-(cyclopropylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 35) 7-(butylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 36) 7-(cyclopentylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 37) 7-(cyclohexylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 38) 7-amino-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 39) 1-methyl-3-(2-methyl-5-(6-(trifluoromethyl)-1H-benzo[d]imidazol-2-yl)phenyl)-7-((6-methylpyridin-3-yl)amino)-3,4-dihydropyrimido[4,5-d]pyrimidin-2(1H)-one;
(Compound No. 40) 3-(5-(5,6-difluoro-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 41) 3-(5-(5,6-dibromo-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 42) 1-methyl-3-(2-methyl-5-(7-methyl-1H-benzo[d]imidazol-2-yl)phenyl)-7-((6-methylpyridin-3-yl)amino)-3,4-dihydropyrimido[4,5-d]pyrimidin-2(1H)-one;
(Compound No. 43) 3-(5-(6-bromo-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 44) 3-(5-(6-chloro-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 45) 1-methyl-3-(2-methyl-5-(6-methyl-1H-benzo[d]imidazol-2-yl)phenyl)-7-((6-methylpyridin-3-yl)amino)-3,4-dihydropyrimido[4,5-d]pyrimidin-2(1H)-one;
(Compound No. 46) 3-(5-(5,6-dichloro-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 47) 3-(5-(5,6-dimethyl-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 48) 1-methyl-3-(2-methyl-5-(6-(trifluoromethyl)-1H-benzo[d]imidazol-2-yl)phenyl)-7-(phenylamino)-3,4-dihydropyrimido[4,5-d]pyrimidin-2(1H)-one;
(Compound No. 49) 1-methyl-3-(2-methyl-5-(6-(trifluoromethyl)-1H-benzo[d]imidazol-2-yl)phenyl)-7-(phenylamino)-3,4-dihydropyrimido[4,5-d]pyrimidin-2(1H)-one;
(Compound No. 50) 7-(cyclopropylamino)-1-methyl-3-(2-methyl-5-(6-(trifluoromethyl)-1H-benzo[d]imidazol-2-yl)phenyl)-3,4-dihydropyrimido[4,5-d]pyrimidin-2(1H)-one;
(Compound No. 51) 7-((2,6-dimethylpyridin-3-yl)amino)-1-methyl-3-(2-methyl-5-(6-(trifluoromethyl)-1H-benzo[d]imidazol-2-yl)phenyl)-3,4-dihydropyrimido[4,5-d]pyrimidin-2(1H)-one;
(Compound No. 52) 1-methyl-3-(2-methyl-5-(6-(trifluoromethyl)-1H-benzo[d]imidazol-2-yl)phenyl)-7-((6-methylpyridin-3-yl)amino)-3,4-dihydropyrimido[4,5-d]pyrimidin-2(1H)-one;
(Compound No. 53) 3-(5-((5,6-difluoro-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one;
(Compound No. 54) 3-(5-((1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one;
(Compound No. 55) 3-(5-((6-bromo-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one;
(Compound No. 56) 3-(5-((6-chloro-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one;
(Compound No. 57) 1-methyl-3-(2-methyl-5-((6-methyl-1H-benzo[d]imidazol-2-yl)amino)phenyl)-7-((6-methylpyridin-3-yl)amino)-3,4-dihydropyrimido[4,5-d]pyrimidin-2(1H)-one;
(Compound No. 58) 3-(5-((6-dibromo-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one;
(Compound No. 59) 3-(5-((6-dichloro-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one;
(Compound No. 60) 3-(5-((5,6-dimethyl-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one;
(Compound No. 61) 3-(5-((5,6-difluoro-1H-benzo[d]imidazol-2-yl)amino)-2-methylphenyl)-1-methyl-7-(phenylamino)-3,4-dihydropyrimido[4,5-d]pyrimidin-2(1H)-one;
(Compound No. 62) 3-(5-((5,6-difluoro-1H-benzo[d]imidazol-2-yl)amino)-2-methylphenyl)-7-((2,6-dimethylpyridin-3-yl)amino)-1-methyl-3,4-dihydropyrimido[4,5-d]pyrimidin-2(1H)-one;
(Compound No. 63) 3-(5-((5,6-difluoro-1H-benzo[d]imidazol-2-yl)amino)-2-methylphenyl)-1-methyl-7-(methylamino)-3,4-dihydropyrimido[4,5-d]pyrimidin-2(1H)-one;
(Compound No. 64) 7-(cyclopropylamino)-3-(5-((5,6-difluoro-1H-benzo[d]imidazol-2-yl)amino)-2-methylphenyl)-1-methyl-3,4-dihydropyrimido[4,5-d]pyrimidin-2(1H)-one; and
(Compound No. 65) 1-methyl-3-(2-methyl-5-((3-(3-(trifluoromethyl)phenyl)oxetan-3-yl)amino)phenyl)-7-((6-methylpyridin-3-yl)amino)-3,4-dihydropyrimido[4,5-d]pyrimidin-2(1H)-one.

The compound of Formula 1 according to the present disclosure may be used in the form of a pharmaceutically acceptable salt derived from an inorganic acid or an organic acid. A preferred salt may be formed with one or more selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

The compound of Formula 1 according to the present disclosure or a pharmaceutically acceptable salt thereof may include a hydrate and a solvate. The hydrate may refer to one formed by the combination of the compound of Formula 1 with a water molecule.

Another aspect of the present disclosure provides a pharmaceutical composition for preventing, alleviating or treating cancer, the pharmaceutical composition containing, as an active ingredient, a compound selected among from the compound of Formula 1 according to the present disclosure, a pharmaceutically acceptable salt thereof, a hydrate thereof, and a stereoisomer thereof.

The pharmaceutical composition according to the present disclosure has excellent ability to inhibit the activity of protein kinase. Specific examples of the protein kinase include ABL1, FGFR2, TAOK2/TAO1, EPHA5, EPHB2, EPHB3, RET, LYN B, EPHA2, FRK/PTK5, EPHA8, LCK, EPHB4, FYN, KHS/MAP4K5, DDR1, EPHA3, P38a/MAPK14, EPHA4, FMS, EPHB1, HCK, FGFR1, ABL2/ARG, EPHA6, c-Src, ACK1, FLT4/VEGFR3, ERBB4/HER4, DDR2, KDR/VEGFR2, LYN, ZAK/MLTK, YES/YES1, BLK, FGR, MLCK2/MYLK2, TAOK1, BMX/ETK, BTK, EPHA1, JAK1, P38b/MAPK11, TIE2/TEK, FLT1/VEGFR1, TXK, SRMS, RAF1, SIK1, MLK3/MAP3K11, PEAK1, TRKA, EPHA7, GLK/MAP4K3, MLK2/MAP3K10, TEC, CSK, TRKC, FES/FPS, SIK2, FGFR3, BRK, YSK4/MAP3K19, ARAF, PDGFRb, TNK1, GCK/MAP4K2, PDGFRa, TNIK, TAK1, ERBB2/HER2, LIMK1, HIPK4, FER, EGFR, JAK2, HPK1/MAP4K1, TRKB, RIPK3, LOK/STK10, LIMK2, MLK1/MAP3K9, BRAF, MEKK3, MEK5, STK32B/YANK2, FGFR4, MEKK2, SLK/STK2, FLT3, PKAcg, TAOK3/JIK, TYRO3/SKY, SIK3, IR, LRRK2, PYK2, NEK11, p70S6K/RPS6KB1, and LATS2.

Thus, the pharmaceutical composition of the present disclosure may be used for the purpose of treating, preventing and alleviating cancer disease caused by abnormal cell growth. Cancer diseases that may be treated, prevented and alleviated by treatment with the pharmaceutical composition according to the present disclosure include stomach cancer, lung cancer, liver cancer, colorectal cancer, small intestine cancer, pancreatic cancer, brain cancer, bone cancer, melanoma, breast cancer, sclerosing adenosis, uterine cancer, cervical cancer, head and neck cancer, esophageal cancer, thyroid cancer, parathyroid cancer, kidney cancer, sarcoma, prostate cancer, urethral cancer, bladder cancer, blood cancer (including leukemia, multiple myeloma, and myelodysplastic syndrome), lymphoma (Hodgkin's disease, and non-Hodgkin's lymphoma), psoriasis, or fibroadenoma.

In particular, the pharmaceutical composition of the present disclosure has inhibitory activity against two kinases, GCK and ACK1, and thus is effective for the treatment of cancer diseases caused by NRAS mutation, for example, melanoma, colorectal cancer, thyroid cancer, or various blood cancers. In addition, the compound represented by Formula 1 exhibits inhibitory activity against the proliferation of the NRAS mutant cell line (OCI-AML3) without showing inhibitory activity against the Ba/F3 (parental) cell line, and thus is particularly effective as a therapeutic agent for treating acute myeloid leukemia (AML).

Preferably, the cancer may be cancer caused by a protein kinase. More preferably, the protein kinase may be at least one selected from among GCK and ACK1.

Another aspect of the present disclosure provides a pharmaceutical composition for preventing, alleviating or treating cancer, the pharmaceutical composition containing, as an active ingredient, any one compound selected from among the above-described compounds.

In another aspect of the present disclosure, there is provided a pharmaceutical composition for preventing, alleviating or treating cancer, wherein the cancer is caused by NRAS mutation.

In another aspect of the present disclosure, there is provided a pharmaceutical composition for preventing, alleviating or treating cancer, wherein the pharmaceutical composition is applied to patients with NRAS mutation.

In another aspect of the present disclosure, there is provided a pharmaceutical composition for preventing, alleviating or treating cancer, wherein the cancer is cancer is at least one selected from the group consisting of melanoma, colorectal cancer, thyroid cancer, and blood cancer.

In another aspect of the present disclosure, there is provided a pharmaceutical composition for preventing, alleviating or treating cancer, wherein the cancer is acute myeloid leukemia (AML).

In another aspect of the present disclosure, there is provided a pharmaceutical composition for preventing, alleviating or treating cancer, wherein the pharmaceutical composition is administered to a patient with NRAS G12D.

The pharmaceutical composition may be applied to experimental animals such as mice, rabbits, rats, guinea pigs, or hamsters, or primates including humans, but is not limited thereto. Preferably, the pharmaceutical composition may be applied to primates including humans, more preferably humans.

In the present disclosure, the term "treating" or "treatment" may be used in a sense including alleviation or amelioration of symptoms, reduction of the range of disease, delay or alleviation of disease progression, amelioration, alleviation or stabilization of disease conditions, partial or complete recovery, prolonging of survival, and other beneficial therapeutic outcomes.

In addition, the treatment of cancer as used in the present specification refers to treatment of all cancer cell types, and the term "cancer" also includes angiogenesis of endothelial cells and mitosis thereof (solid tumors, tumor metastases and benign tumors). Examples of the cancer include, but are not limited to, breast cancer, ovarian cancer, cervical cancer, prostate cancer, testicular cancer, genitourinary tract cancer, esophageal cancer, laryngeal cancer, glioblastoma, stomach cancer, skin cancer, keratoacanthoma, lung cancer, squamous cell carcinoma, large cell carcinoma, small cell carcinoma, lung adenocarcinoma, bone cancer, colon cancer, adenoma, pancreatic cancer, adenocarcinoma, thyroid cancer, follicular adenocarcinoma, undifferentiated cancer, papillary cancer, seminoma, melanoma, sarcoma, bladder cancer, liver and bile duct cancer, kidney cancer, myeloid disease, lymphoid disease, Hodgkin's disease, hair cell cancer, oral cancer, pharyngeal (laryngeal) cancer, lip cancer, tongue cancer, small intestine cancer, colorectal cancer, large intestine cancer, rectal cancer, brain cancer, central nervous system cancer, leukemia, angioma, trachoma, or pyogenic granuloma.

Depending on the aspect and method of use of the pharmaceutical composition of the present disclosure, the content of the compound represented by Formula 1, a pharmaceutically acceptable salt thereof, or a hydrate thereof, which is an active ingredient, may be appropriately selected and adjusted by those skilled in the art.

For example, the pharmaceutical composition may contain the compound represented by Formula 1, a pharmaceutically acceptable salt thereof, or a hydrate thereof in an amount of 0.1 to 10 wt%, more preferably 0.5 to 5 wt% by weight, based on the total weight of the composition.

The compound represented by Formula 1, a pharmaceutically acceptable salt thereof, or a hydrate thereof may be contained alone in the pharmaceutical composition or may also be contained together with a pharmaceutically acceptable carrier, excipient, diluent or accessory ingredient.

Examples of the pharmaceutically acceptable carrier, excipient or diluent include, but are not limited to, at least one selected from the group consisting of lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, dextrin, calcium carbonate, propylene glycol, liquid paraffin, and physiological saline. In addition, any conventional carrier, excipient or diluent may also be used. In addition, the pharmaceutical composition may further contain a conventional filler, extender, binder, disintegrant, anti-aggregating agent, lubricant, wetting agent, pH-adjusting agent, nutrient, vitamin, electrolyte, alginic acid and its salt, pectic acid and its salt, protective colloid, glycerin, fragrance, emulsifier or preservative.

The compound of Formula 1 according to the present disclosure or a pharmaceutically acceptable salt may be co-administered with other anticancer drugs for treating cancer or tumors, thus enhancing the therapeutic effects of the anticancer drugs.

Specifically, the pharmaceutical composition may further contain one or more other anticancer drugs or other therapeutic agents known to be effective for the treatment or prevention of cancer, in addition to the active ingredient, and may be used in combination therapy in which they are applied simultaneously or at different times. For example, other anticancer drugs or other therapeutic agents that may be applied to the combination therapy may include, but are not limited to, one or more compounds selected from the group consisting of Gleevec^{®} (imatinib), Sutent^{®} (sunitinib), Herceptin^{®} (Trastuzumab), Velcade^{®} (Bortezomib), dexamethasone, Nexavar^{®} (Sorafenib), aromatase inhibitors, and kinase inhibitors.

The mode of administration of the pharmaceutical composition may be oral or parenteral. For example, the pharmaceutical composition may be administered through various routes, including oral, transdermal, subcutaneous, intravenous or intramuscular routes. In addition, the formulation of the composition may vary depending on the method of use thereof, and the composition may be formulated using a method well known in the art to which the present disclosure pertains, so as to provide rapid, sustained or delayed release of the active ingredient after administration to a mammal. In general, solid preparations for oral administration include tablets, troches, soft or hard capsules, pills, powders, and granules, and these formulations may be prepared by mixing one or more excipients, for example, starch, calcium carbonate, sucrose, lactose, or gelatin. In addition to simple excipients, lubricants such as magnesium stearate and talc may also be used. Liquid formulations for oral administration include suspensions, liquids for internal use, emulsions, and syrups, which may contain various excipients, for example, wetting agents, sweetening agents, fragrances, preservatives, and the like, in addition to water and liquid paraffin, which are commonly used simple diluents. Dosage forms for parenteral administration include cream, lotions, ointments, plasters, liquids, solutions, aerosols, fluid extracts, elixirs, infusions, sachets, patches, or injections. When the dosage form for parenteral administration is a dosage form for injection, it may preferably be in the form of an isotonic aqueous solution or suspension.

The pharmaceutical composition may further contain a sterilizing agent, a preservative, a stabilizer, a wetting agent or an emulsifying agent, adjuvants such as a salt and/or buffer for regulating osmotic pressure, and other therapeutically useful substances, and may be formulated according to a conventional mixing, granulation or coating method. In addition, the pharmaceutical composition may be formulated using an appropriate method known in the art.

In addition, the dosage of the pharmaceutical composition may be determined in consideration of the mode of administration, the patient's age and sex, the patient's disease severity and condition, the *in vivo* absorption rate of the active ingredient, the rate of inactivation, and drugs to be used in combination, and may be administered once or several times. The active ingredient of the pharmaceutical composition may preferably administered to mammals including humans once or several times a day by an oral or parenteral route at a dose of 0.001 to 100 mg/kg body weight/day, preferably 0.01 to 35 mg/kg body weight/day.

Another embodiment of the present disclosure provides a method for treating cancer, the method comprising a step of administering a therapeutically effective amount of the compound represented by Formula 1, a pharmaceutically acceptable salt thereof, or a hydrate thereof.

Preferably, the treatment method may further comprise a step of identifying a patient in need of the prevention or treatment of the cancer, before the administering step.

In the present disclosure, the term "therapeutically effective amount" refers to an amount of the active ingredient for a mammal, which is effective for the prevention or treatment of cancer. The therapeutically effective amount may be adjusted depending on various factors, including the kind of disease, the severity of the disease, the kinds and contents of the active ingredient and other ingredients contained in the composition, the type of formulation, the patient's age, weight, general health status, sex and diet, the time of administration, the route of administration, the blood clearance of the composition, the duration of treatment, and drugs that are used concurrently. Preferably, as described above, the active ingredient may be administered once or several times a day by an oral or parenteral route at a dose of 0.001 to 100 mg/kg body weight/day, preferably 0.01 to 35 mg/kg body weight/day.

In addition, the present disclosure is directed to a method for producing the 7-amino-3,4-dihydropyrimidopyrimidin-2-one derivative compound represented by Formula 1, a pharmaceutically acceptable salt thereof, or a hydrate thereof.

Meanwhile, the present disclosure is characterized by a method for producing the compound represented by Formula 1. The production method according to the present disclosure will now be described in detail.

In one embodiment, the compound represented by Formula 1 according to the present disclosure may be produced by any one or more of the following Production Methods 1 to 4:

### Production Method 1

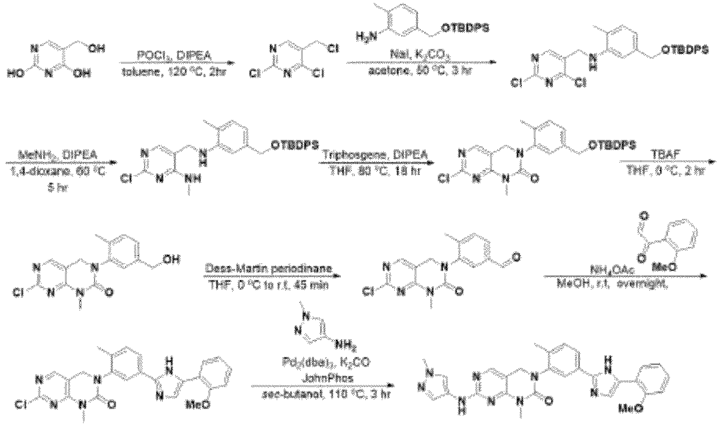

### Production Method 2

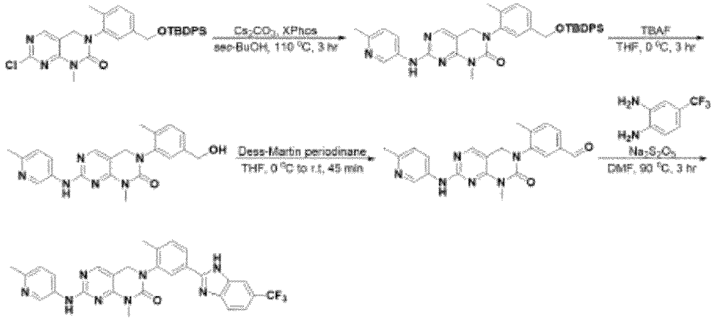

### Production Method 3

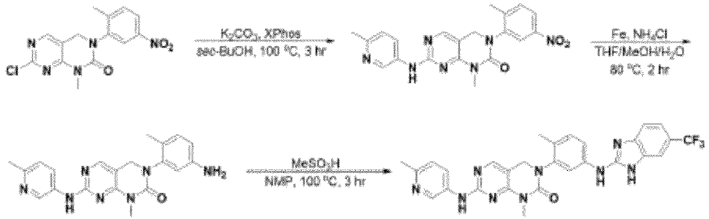

### Production Method 4

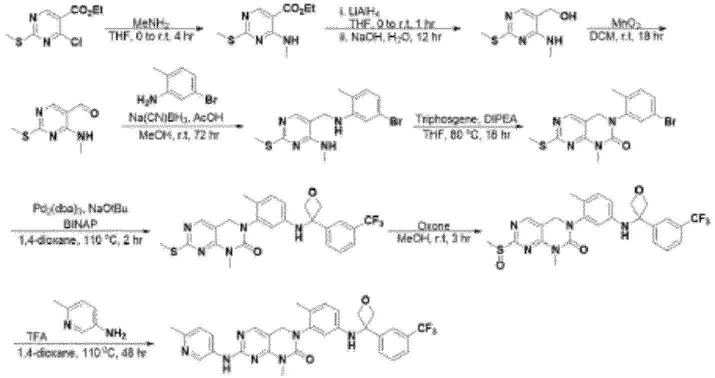

### Mode for Invention

Hereinafter, the present disclosure will be described in detail with reference to synthesis examples, examples and experimental examples. However, the following synthesis examples, examples and experimental examples serve to merely illustrate the present disclosure, and the scope of the present disclosure is not limited by the following examples.

### [Representative Synthesis Example 1]

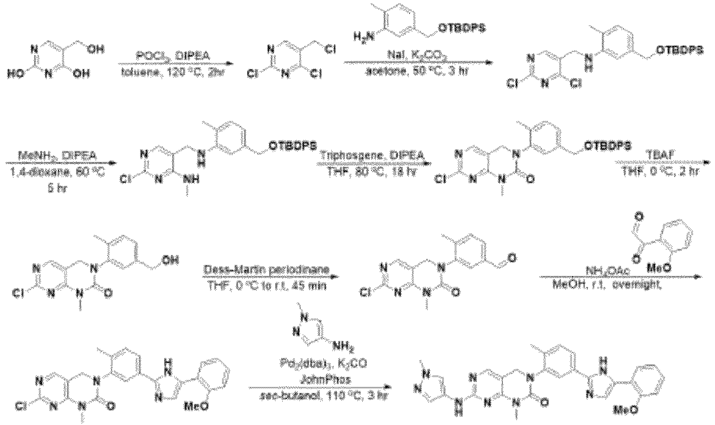

### Example 1: 3-(5-(5-(2-methoxyphenyl)-1H-imidazol-2-yl)-2-methylphenyl)-1-methyl-7-((1-methyl-1H-pyrazol-4-yl)amino)-3,4-dihydropyrimido[4,5-d]pyrimidin-2(1H)-one

### Step 1-1) 2,4-dichloro-5-(chloromethyl)pyrimidine

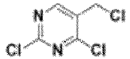

5-(hydroxymethyl)pyrimidine-2,4-diol (8.0 g, 56.32 mmol) and phosphorus oxychloride (POCl₃) (26.3 mL, 281.47 mmol) were added to toluene (180 mL), and then gas contained in the mixture solution was removed by injecting nitrogen gas for 5 minutes. Thereafter, the reaction vessel was placed and cooled in an ice water bath at a 0°C, and N,N-diisopropylethylamine (29.4 mL, 168.88 mmol) was slowly added thereto dropwise, followed by stirring under reflux at 120°C for 2 hours. After completion of the reaction, the mixture solution was cooled to room temperature and water was added thereto dropwise. The resulting solution was extracted with ethyl acetate, dried with sodium sulfate, and then concentrated under reduced pressure. The resulting residue was purified by MPLC (high-performance automatic separation and purification system/ethyl acetate: hexane = 1: 30 (v/v)), and the resulting solution was concentrated under reduced pressure to obtain 9.3 g (84% yield) of the title compound as a white solid.

LCMS (ESI+, m/z): 196.95 [M+H]+

¹H NMR (400 MHz, CDCl₃) δ 8.66 (s, 1H), 4.64 (s, 2H)

### Step 1-2) 5-(((tert-butyldiphenylsilyl)oxy)methyl)-2-methylaniline

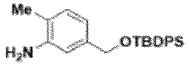

3-amino-4-methylbenzyl alcohol (3.0 g, 21.87 mmol) was dissolved in anhydrous dry N,N-dimethylformamide (30 mL) and cooled to 0°C. Tert-butylchlorodiphenylsilane (6.0 g, 21.87 mmol) and imidazole (3.72 g, 54.67 mmol) were added thereto, followed by stirring at room temperature for 18 hours. After completion of the reaction, the mixture solution was cooled to room temperature and water was added thereto dropwise, followed by extraction with diethyl ether. The combined organic layers were dried with anhydrous sodium sulfate and concentrated. The resulting residue was purified by MPLC (ethyl acetate: hexane = 1: 15 (v/v)), and the resulting solution was concentrated under reduced pressure to obtain 7.8 g (95% yield) of the title compound as a white solid.

LCMS(ESI+, m/z): 376.60 [M+H]+

¹H NMR (400 MHz, CDCl₃) δ 7.81 - 7.67 (m, 4H), 7.42 (dt, *J* = 13.5, 6.5 Hz, 6H), 7.04 (d, *J* = 7.8 Hz, 1H), 6.72 (d, *J* = 6.3 Hz, 2H), 4.72 (s, 2H), 2.19 (s, 3H), 1.13 (s, 9H) .

### Step 1-3) 5-(((tert-butyldiphenylsilyl)oxy)methyl)-N-((2,4-dichloropyrimidin-5-yl)methyl)-2-methylaniline

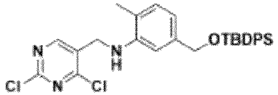

2, 4-dichloro-5- (chloromethyl)pyrimidine (2.0 g, 10.2 mmol) produced in Step 1-1 of Example 1 and 5-(((tert-butyldiphenylsilyl)oxy)methyl)-2-methylaniline (4.95 g, 13.2 mmol) produced in Step 1-2 of Example 1 were dissolved in acetone (10 mL), and then sodium iodide (1.82 g, 12.2 mmol) and potassium carbonate (2.10 g, 15.2 mmol) were added thereto, followed by stirring under reflux at 50°C for 3 hours. After completion of the reaction, the mixture solution was cooled to room temperature, filtered through a celite pad, and then concentrated using a vacuum distillation device. The concentrate was diluted in dichloromethane and extracted with dichloromethane and water. The combined organic layers were dried with sodium sulfate and concentrated. The resulting residue was purified by MPLC (ethyl acetate: hexane = 1: 20 (v/v)), and the resulting solution was concentrated under reduced pressure to obtain 4.06 g (74% yield) of the title compound as a white solid.

LCMS(ESI+, m/z) : 536.25 [M+H]+

¹H NMR (400 MHz, CDCl₃) δ 8.47 (s, 1H), 7.63 (dt, J = 6.6, 1.6 Hz, 4H), 7.46 - 7.33 (m, 6H), 7.06 (d, *J* = 7.6 Hz, 1H), 6.65 (dd, *J* = 7.5, 1.5 Hz, 1H), 6.50 (d, *J* = 1.6 Hz, 1H), 4.67 (s, 2H), 4.49 (s, 2H), 2.23 (s, 3H), 1.00 (s, 9H).

### Step 1-4) 5-(((5-(((tert-butyldiphenylsilyl)oxy)methyl)-2-methylphenyl)amino)methyl)-2-chloro-N-methylpyrimidine-4-amine

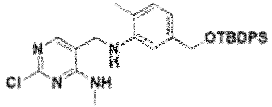

5-(((tert-butyldiphenylsilyl)oxy)methyl)-N-((2,4-dichloropyrimidin-5-yl)methyl)-2-methylaniline (3.41 g, 6.37 mmol) produced in Step 1-3 of Example 1 was dissolved in 1,4-dioxane (60 mL). 33% methylamine (methanol solution, 1.58 mL, 12.71 mmol) and N,N-diisopropylethylamine (3.32 mL, 19.06 mmol) were slowly added thereto dropwise, followed by stirring under reflux at 60°C for 5 hours. After completion of the reaction, the mixture solution was cooled to room temperature and water was added thereto dropwise, followed by extraction with dichloromethane. The combined organic layers were dried with anhydrous sodium sulfate and concentrated. The resulting residue was purified by MPLC (ethyl acetate: hexane = 1: 4 (v/v)), and the resulting solution was concentrated under reduced pressure to obtain 2.1 g (62% yield) of the title compound as a white solid.

LCMS (ESI+, m/z) : 531.45 [M+H]+

¹H NMR (400 MHz, CDCl₃) δ 7.91 (s, 1H), 7.76 - 7.65 (m, 4H), 7.49 - 7.33 (m, 6H), 7.08 (d, *J* = 7.4 Hz, 1H), 6.80 (d, *J* = 7.9 Hz, 2H), 6.16 (d, *J* = 5.4 Hz, 1H), 4.75 (s, 2H), 4.08 (s, 2H), 3.03 (d, *J* = 4.9 Hz, 3H), 2.14 (s, 3H), 1.10 (s, 9H).

### Step 1-5) 3-(5-(((tert-butyldiphenylsilyl)oxy)methyl)-2-methylphenyl)-7-chloro-1-methyl-3,4-dihydropyrimido[4,5-d]pyrimidin-2(1H)-one

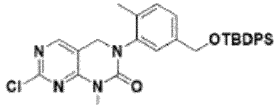

5-(((5-(((tert-butyldiphenylsilyl)oxy)methyl)-2-methylphenyl)amino)methyl)-2-chloro-N-methylpyrimidine-4-amine (1.95 g, 3.67 mmol) produced in Step 1-4 of Example 1 was dissolved in anhydrous dry tetrahydrofuran (THF, 40 mL) and cooled to 0°C. Triphosgene (0.612 mL, 3.67 mmol) and N,N-diisopropylethylamine (3.20 ml, 18.35 mmol) were sequentially slowly added dropwise thereto, followed by stirring under reflux for 1 hour at room temperature and then at 80 °C for 18 hours. After completion of the reaction, the mixture solution was cooled to room temperature and a saturated aqueous solution of sodium hydrogen carbonate was added thereto dropwise, followed by extraction with ethyl acetate. The combined organic layers were dried with anhydrous sodium sulfate and concentrated. The resulting residue was purified by MPLC (ethyl acetate: hexane = 1: 5 (v/v)), and the resulting solution was concentrated under reduced pressure to obtain 1.5 g (74% yield) of the title compound as a white solid.

LCMS (ESI+, m/z) : 557.20 [M+H]+

¹H NMR (400 MHz, CDCl₃) δ 8.09 (d, *J* = 1.2 Hz, 1H), 7.67 (ddt, *J* = 5.7, 4.0, 1.6 Hz, 4H), 7.48 - 7.32 (m, 6H), 7.26 (d, *J* = 1.1 Hz, 2H), 7.15 (s, 1H), 4.75 (s, 2H), 4.69 (dd, *J* = 14.9, 1.2 Hz, 1H), 4.50 (dd, *J* = 14.9, 1.0 Hz, 1H), 3.46 (s, 3H), 2.21 (s, 3H), 1.09 (s, 9H).

### Step 1-6) 7-chloro-3-(5-(hydroxymethyl)-2-methylphenyl)-1-methyl-3,4-dihydropyrimido[4,5-d]pyrimidin-2(1H)-one

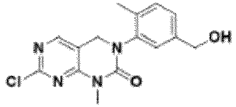

3-(5-(((tert-butyldiphenylsilyl)oxy)methyl)-2-methylphenyl)-7-chloro-1-methyl-3,4-dihydropyrimido[4,5-d]pyrimidin-2(1H)-one (1 g, 1.80 mmol) produced in Step 1-5 of Example 1 was dissolved in anhydrous dry tetrahydrofuran (50 mL), and the reaction vessel was placed and cooled in an ice water bath at a 0°C. 1 M tetrabutylammonium fluoride (tetrahydrofuran solution, 5.38 mL, 5.38 mmol) was added thereto dropwise, followed by stirring at 0°C for 2 hours. After completion of the reaction, a saturated aqueous solution of ammonium chloride was added dropwise to the mixture solution, followed by warming to room temperature and extraction with ethyl acetate. The combined organic layers were dried with anhydrous sodium sulfate and concentrated. The resulting residue was purified by high-performance automatic separation and purification system (ethyl acetate: hexane = 1: 2 (v/v)), and the resulting solution was concentrated under reduced pressure to obtain 569 mg (100% yield) of the title compound as a white solid.

LCMS (ESI+, m/z) : 319.05 [M+H]+

¹H NMR (400 MHz, CDCl₃) δ 7.95 (s, 1H), 7.23 - 7.12 (m, 3H), 4.67 (d, *J* = 14.7 Hz, 1H), 4.47 (d, *J* = 14.7 Hz, 1H), 4.50 (s, 2H), 4.08 (d, *J* = 4.9 Hz, 1H), 3.35 (s, 3H), 2.15 (s, 3H).

### Step 1-7) 3-(7-chloro-1-methyl-2-oxo-1,4-dihydropyrimido[4,5-d]pyrimidin-3(2H)-yl)-4-methylbenzaldehyde

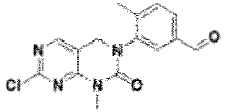

7-chloro-3-(5-(hydroxymethyl)-2-methylphenyl)-1-methyl-3,4-dihydropyrimido[4,5-d]pyrimidin-2(1H)-one (1.1 g, 3.45 mmol) produced in Step 1-6 of Example 1 was dissolved in anhydrous dry tetrahydrofuran (30 mL) and cooled to 0°C. Dess-Martin Periodinane (2.9 g, 6.83 mmol) was added thereto, followed by stirring at room temperature for 45 minutes. After completion of the reaction, a saturated aqueous solution of sodium hydrogen carbonate was added dropwise to the mixture solution, followed by extraction with dichloromethane. The combined organic layers were washed with brine, dried with anhydrous sodium sulfate, and then concentrated. The resulting residue was purified by MPLC (ethyl acetate: hexane = 1: 3 (v/v)), and the resulting solution was concentrated under reduced pressure to obtain 721 mg (66% yield) of the title compound as a white solid.

LCMS (ESI+, m/z) : 317.0 [M+H]+

¹H NMR (400 MHz, CDCl₃) δ 9.96 (s, 1H), 8.14 (s, 1H), 7.79 (dd, *J* = 4.1, 2.4 Hz, 2H), 7.49 (d, *J* = 8.2 Hz, 1H), 4.86 (d, *J* = 14.7 Hz, 1H), 4.58 (d, *J* = 14.7 Hz, 1H), 3.46 (s, 3H), 2.32 (s, 3H) .

### Step 1-8) 2-(2-methoxyphenyl)-2-oxoacetaldehyde

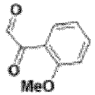

Selenium dioxide (1.1 g, 9.91 mmol) is added to water (1.0 mL), and the solution was added dropwise to 1,4-dioxane (9.0 mL) and then stirred at 50 to 55°C until the selenium dioxide was completely dissolved. After the selenium dioxide was dissolved, 2'-methoxyacetophenone (1.0 g, 6.65 mmol) was added thereto dropwise at 50 to 55°C, followed by stirring under reflux at an elevated temperature of 110°C for 12 hours. After completion of the reaction, the mixture solution was cooled to room temperature, filtered through a celite pad, and then concentrated using a vacuum distillation device. Water was added to the concentrate, followed by extraction with ethyl acetate. Then, the combined organic layers were dried with anhydrous sodium sulfate and concentrated using a vacuum distillation device.

LCMS (ESI+, m/z) : 165.15 [M+H]+

### Step 1-9) 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one

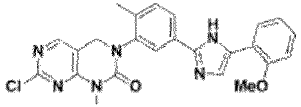

3-(7-chloro-1-methyl-2-oxo-1,4-dihydropyrimido[4,5-d]pyrimidine-3(2H)-yl)-4-methylbenzaldehyde (350 mg, 1.10 mmol) produced in Step 1-7 of Example 1 was dissolved in methanol (3.0 mL), and ammonium acetate (250 mg, 3.24 mmol) was added thereto, followed by stirring at room temperature for 30 minutes. Then, 2-(2-methoxyphenyl)-2-oxoacetaldehyde (360 mg, 2.18 mmol) produced in Step 1-8 of Example 1 was dissolved in methanol (1.0 mL) and slowly added thereto dropwise, followed by stirring at room temperature for 12 hours. After completion of the reaction, the mixture solution was concentrated using a vacuum distillation device. The resulting residue was purified by MPLC (dichloromethane: ethyl acetate = 1: 3 (v/v)), and the resulting solution was concentrated under reduced pressure to obtain 220 mg (43% yield) of the title compound as a white solid.

LCMS (ESI+, m/z) : 461.10 [M+H]+

¹H NMR (400 MHz, MeOD) δ 8.27 (s, 1H), 7.93 (d, *J* = 1.9 Hz, 1H), 7.91 (d, *J* = 7.4 Hz, 1H), 7.88 (dd, *J* = 7.9, 1.9 Hz, 1H), 7.59 (s, 1H), 7.46 (d, *J* = 8.1 Hz, 1H), 7.28 (ddd, *J* = 8.8, 7.3, 1.7 Hz, 1H), 7.09 (d, *J* = 8.3 Hz, 1H), 7.03 (td, *J* = 7.5, 1.1 Hz, 1H), 4.95 (d, *J* = 15.0 Hz, 1H), 4.75 (d, *J* = 15.0 Hz, 1H), 3.97 (s, 3H), 3.45 (s, 3H), 2.30 (s, 3H).

### Step 1-10) 3-(5-(5-(2-methoxyphenyl)-1H-imidazol-2-yl)-2-methylphenyl)-1-methyl-7-((1-methyl-1H-pyrazol-4-yl)amino)-3,4-dihydropyrimido[4,5-d]pyrimidin-2(1H)-one

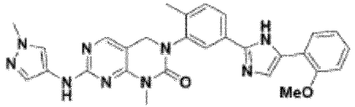

7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one (50 mg, 0.108 mmol) produced in Step 1-9 of Example 1 was dissolved in 2-butanol (5.0 mL). Potassium carbonate (17.6 mg, 0.127 mmol), 4-amino-1-methylpyrazole (16 mg, 0.163 mmol), JohnPhos (6.5 mg, 0.022 mmol) and Pd₂(dba)₃ (20 mg, 0.022 mmol) were added thereto, followed by stirring under reflux at 110°C for 3 hours. After completion of the reaction, the reaction solution was filtered through a celite pad, and the filtrate was concentrated using a vacuum distillation device. The resulting residue was purified by HPLC (high-performance liquid chromatography/acetonitrile: water = 5: 95 → 95:5 (v/v), 40 minutes), and the resulting solution was freeze-dried to obtain 20.5 mg (36% yield) of the title compound as a white solid.

LCMS (ESI+, m/z) : 522.20 [M+H]+

¹H NMR (400 MHz, MeOD) δ 7.94 (s, 1H), 7.90 (d, *J* = 2.0 Hz, 1H), 7.81 (t, *J* = 4.3 Hz, 3H), 7.67 (dd, *J* = 7.7, 1.7 Hz, 1H), 7.57 - 7.50 (m, 2H), 7.39 (ddd, *J* = 8.8, 7.4, 1.7 Hz, 1H), 7.12 (d, *J* = 8.4 Hz, 1H), 7.03 (t, *J* = 7.6 Hz, 1H), 4.70 (d, *J* = 14.2 Hz, 2H), 4.54 (d, *J* = 14.2 Hz, 2H), 3.90 (s, 3H), 3.80 (s, 3H), 3.40 (s, 3H), 2.27 (s, 3H).

### Example 2: 7-((2,6-dimethylpyridin-3-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one

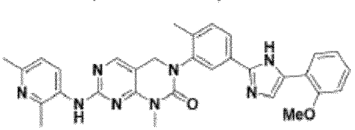

20.1 mg (34% yield) of the title compound was obtained by repeating the procedure of Step 1-10 of Example 1, except that 3-amino-2,6-dimethylpyridine (19.8 mg, 0.163 mmol) was used instead of 4-amino-1-methylpyrazole in Step 1-10 of Example 1.

LCMS (ESI+, m/z) : 547.25 [M+H]+

¹H NMR (400 MHz, MeOD) δ 8.84 (d, *J* = 8.7 Hz, 1H), 8.13 (s, 1H), 7.99 (d, *J* = 2.0 Hz, 1H), 7.92 - 7.88 (m, 2H), 7.76 (dd, *J* = 7.7, 1.7 Hz, 1H), 7.70 (d, *J* = 8.6 Hz, 1H), 7.64 (d, *J* = 8.1 Hz, 1H), 7.48 (ddd, *J* = 8.7, 7.4, 1.7 Hz, 1H), 7.21 (d, *J* = 8.2 Hz, 1H), 7.12 (td, *J* = 7.6, 1.0 Hz, 1H), 4.86 (d, *J* = 14.1 Hz, 2H), 4.66 (d, *J* = 14.1 Hz, 2H), 3.99 (s, 3H), 3.40 (s, 3H), 2.73 (s, 3H), 2.73 (s, 3H), 2.36 (s, 3H).

### Example 3: 3-(5-(5-(2-methoxyphenyl)-1H-imidazol-2-yl)-2-methylphenyl)-1-methyl-7-((6-methylpyridin-3-yl)amino)-3,4-dihydropyrimido[4,5-d]pyrimidin-2(1H)-one

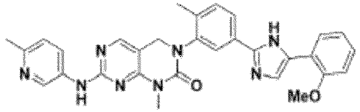

18.5 mg (35% yield) of the title compound was obtained by repeating the procedure of Step 1-10 of Example 1, except that 6-methylpyridine-3-amine (17.6 mg, 0.163 mmol) was used instead of 4-amino-1-methylpyrazole in Step 1-10 of Example 1.

LCMS (ESI+, m/z) : 533.05 [M+H]+

¹H NMR (400 MHz, MeOD) δ 9.45 (t, *J* = 1.8 Hz, 1H), 8.46 (dt, *J* = 8.8, 1.8 Hz, 1H), 8.25 (s, 1H), 8.00 (s, 1H), 7.92 (d, *J* = 8.9 Hz, 2H), 7.78 (dd, *J* = 8.6, 2.8 Hz, 2H), 7.66 (d, *J* = 8.1 Hz, 1H), 7.49 (t, *J* = 7.8 Hz, 1H), 7.22 (d, J = 8.4 Hz, 1H), 7.13 (t, *J* = 7.6 Hz, 1H), 4.87 (d, *J* = 14.2 Hz, 2H), 4.71 (d, *J* = 14.2 Hz, 2H), 4.00 (s, 3H), 3.50 (s, 3H), 2.71 (s, 3H), 2.37 (s, 3H).

### Example 4: 7-((1-(1-ethylpiperidin-4-yl)-1H-pyrazol-4-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one

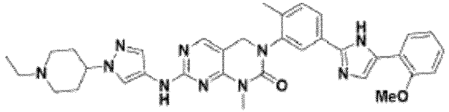

17.4 mg (26% yield) of the title compound was obtained by repeating the procedure of Step 1-10 of Example 1, except that 1-(1-ethylpiperidin-4-yl)-1H-pyrazole-4-amine (31.6 mg, 0.163 mmol) was used instead of 4-amino-1-methylpyrazole in Step 1-10 of Example 1.

LCMS (ESI+, m/z) : 673.30 [M+H]+

¹H NMR (400 MHz, MeOD) δ 8.06 (s, 1H), 8.03 (s, 1H), 8.01 (d, *J* = 2.0 Hz, 1H), 7.93 - 7.89 (m, 2H), 7.77 (dd, J = 7.7, 1.7 Hz, 1H), 7.71 (s, 1H), 7.65 (d, *J* = 8.2 Hz, 1H), 7.49 (ddd, *J* = 8.8, 7.4, 1.7 Hz, 1H), 7.22 (dd, *J* = 8.5, 1.0 Hz, 1H), 7.13 (td, *J* = 7.6, 1.0 Hz, 1H), 4.82 (d, *J* = 14.2 Hz, 2H ), 4.64 (d, *J* = 14.2 Hz, 2H), 4.54 (tt, *J* = 10.6, 4.6 Hz, 1H), 4.00 (s, 3H), 3.75 (d, *J* = 12.7 Hz, 2H), 3.50 (s, 3H), 3.25 (q, *J* = 7.3 Hz, 2H), 3.22 - 3.12 (m, 2H), 2.37 (s, 3H), 2.42-2.26 (m, 4H), 1.39 (t, *J* = 7.3 Hz, 3H).

### Example 5: 7-((6-(1-ethylpiperidin-4-yl)pyridin-3-yl)amino)-3-(5-(5-(2-methoxyphenyl)-1H-imidazole-2-yl)-2-methylphenyl)-1-methyl-3,4-dihydropyrimido[4,5-d]pyrimidin-2(1H)-one

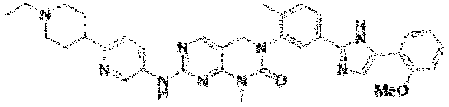

44.1 mg (70% yield) of the title compound was obtained by repeating the procedure of Step 1-10 of Example 1, except that 6-(1-ethylpiperidin-4-yl)pyridine-3-amine (33.4 mg, 0.163 mmol) was used instead of 4-amino-1-methylpyrazole in Step 1-10 of Example 1.

LCMS (ESI+, m/z) : 630.30 [M+H]+

¹H NMR (400 MHz, MeOD) δ 9.25 (d, *J* = 2.6 Hz, 1H), 8.39 (dd, *J* = 8.7, 2.6 Hz, 1H), 8.20 (s, 1H), 8.02 (d, *J* = 2.0 Hz, 1H), 7.93 - 7.89 (m, 2H), 7.77 (dd, *J* = 7.7, 1.7 Hz, 1H), 7.63 (dd, *J* = 8.5, 6.3 Hz, 2H), 7.49 (ddd, *J* = 8.8, 7.4, 1.7 Hz, 1H), 7.21 (d, *J* = 8.3 Hz, 1H), 7.12 (td, *J* = 7.6, 1.0 Hz, 1H), 4.89 (d, *J* = 14.2 Hz, 2H), 4.68 (d, *J* = 14.2 Hz, 2H), 4.00 (s, 3H), 3.79 - 3.70 (m, 2H), 3.49 (s, 3H), 3.29 - 3.20 (m, 3H), 3.14 (td, *J* = 12.8, 2.9 Hz, 2H), 2.37 (s, 3H), 2.33 - 2.23 (m, 2H), 2.15 (tt, *J* = 13.1, 6.7 Hz, 2H), 1.40 (t, *J* = 7.3 Hz, 3H).

### Example 6: 7-((6-(4-acetylpiperazin-1-yl)pyridin-3-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one

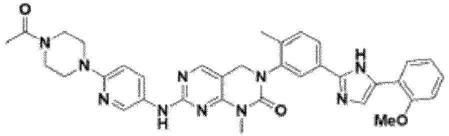

25.3 mg (39% yield) of the title compound was obtained by repeating the procedure of Step 1-10 of Example 1, except that 1-(4-(5-aminopyridin-2-yl)piperazin-1-yl)ethan-1-one (35.8 mg, 0.163 mmol) was used instead of 4-amino-1-methylpyrazole in Step 1-10 of Example 1.

LCMS (ESI+, m/z) : 645.30 [M+H]+

¹H NMR (400 MHz, MeOD) δ 8.79 (d, *J* = 2.7 Hz, 1H), 8.16 (s, 1H), 8.13 (dd, *J* = 9.6, 2.7 Hz, 1H), 7.99 (d, *J* = 2.0 Hz, 1H), 7.91 (d, *J* = 6.5 Hz, 2H), 7.77 (dd, *J* = 7.7, 1.7 Hz, 1H), 7.66 (d, *J* = 8.1 Hz, 1H), 7.49 (ddd, *J* = 8.8, 7.4, 1.6 Hz, 1H), 7.33 (d, *J* = 9.7 Hz, 1H), 7.22 (dd, *J* = 8.4, 1.0 Hz, 1H), 7.13 (td, *J* = 7.6, 1.0 Hz, 1H), 4.90 (d, *J* = 14.1 Hz, 2H), 4.67 (d, *J* = 14.1 Hz, 2H), 4.00 (s, 3H), 3.79 (dd, *J* = 6.5, 3.8 Hz, 4H), 3.74 (dd, *J* = 7.1, 4.0 Hz, 2H), 3.67 (dd, *J* = 6.7, 4.2 Hz, 2H), 3.48 (s, 3H), 2.37 (s, 3H), 2.17 (s, 3H).

### Example 7: 7-((1-(1-acetylpiperidin-4-yl)-1H-pyrazol-4-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one

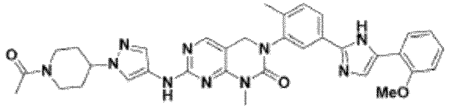

19.8 mg (31% yield) of the title compound was obtained by repeating the procedure of Step 1-10 of Example 1, except that 1-(4-(4-amino-1H-pyrazol-1-yl)piperidin-1-yl)ethan-1-one (33.9 mg, 0.163 mmol) was used instead of 4-amino-1-methylpyrazole in Step 1-10 of Example 1.

LCMS (ESI+, m/z) : 633.30 [M+H]+

¹H NMR (400 MHz, MeOD) δ 8.06 (s, 1H), 7.99 (d, *J* = 0.8 Hz, 1H), 7.97 (d, *J* = 2.0 Hz, 1H), 7.90 (d, *J* = 7.8 Hz, 2H), 7.76 (dd, *J* = 7.8, 1.7 Hz, 1H), 7.67 (s, 1H), 7.65 (s, 1H), 7.50 (ddd, *J* = 8.9, 7.5, 1.7 Hz, 1H), 7.22 (dd, *J* = 8.6, 1.0 Hz, 1H), 7.13 (td, *J* = 7.6, 1.0 Hz, 1H), 4.85 (d, *J* = 14.0 Hz, 2H), 4.64 (d, *J* = 14.0 Hz, 2H), 4.51 - 4.38 (m, 1H), 4.07 (d, *J* = 14.3 Hz, 2H), 4.00 (s, 3H), 3.49 (s, 3H), 2.92 - 2.77 (m, 2H), 2.38 (s, 3H), 2.16 (s, 3H), 1.97 (dtd, *J* = 41.7, 11.8, 4.4 Hz, 4H).

### Example 8: 7-((2,6-dimethylpyridin-3-yl)amino)-1-methyl-3-(2-methyl-5-(5-(2-(trifluoromethoxy)phenyl)-1H-imidazole-2-yl)phenyl)-3,4-dihydropyrimido[4,5-d]pyrimidin-2(1H)-one

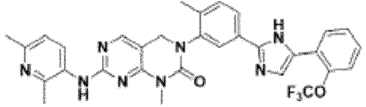

36.9 mg (61% yield) of the title compound was obtained by repeating the procedure of Step 1-10 of Example 1, except that, in Step 1-10 of Example 1, 7-chloro-1-methyl-3-(2-methyl-5-(5-(2-(trifluoromethoxy)phenyl-1H-imidazol-2-yl)phenyl)-3,4-dihydropyrimido[4,5-d]pyrimidin-2(1H)-one (55.5 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 3-amino-2,6-dimethylpyridine (19.8 mg, 0.163 mmol) was used instead of 4-amino-1-methylpyrazole.

LCMS (ESI+, m/z) : 601.20 [M+H]+

¹H NMR (400 MHz, MeOD) δ 8.83 (d, *J* = 8.7 Hz, 1H), 8.14 (s, 1H), 7.98 (d, *J* = 2.0 Hz, 1H), 7.94 (dd, *J* = 7.5, 1.9 Hz, 1H), 7.90 (dd, *J* = 8.0, 2.0 Hz, 1H), 7.79 (s, 1H), 7.70 (d, *J* = 8.7 Hz, 1H), 7.62 (d, *J* = 8.2 Hz, 1H), 7.59 - 7.50 (m, 3H), 4.87 (d, *J* = 14.3 Hz, 2H), 4.66 (d, *J* = 14.3 Hz, 2H), 3.41 (s, 3H), 2.74 (s, 3H), 2.73 (s, 3H), 2.35 (s, 3H) .

### Example 9: 1-methyl-3-(2-methyl-5-(5-(2-(trifluoromethoxy)phenyl)-1H-imidazol-2-yl)phenyl)-7-((6-methylpyridin-3-yl)amino)-3,4-dihydropyrimido[4,5-d]pyrimidin-2(1H)-one

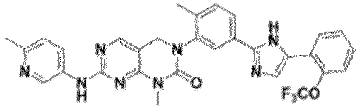

43.2 mg (68% yield) of the title compound was obtained by repeating the procedure of Step 1-10 of Example 1, except that, in Step 1-10 of Example 1, 7-chloro-1-methyl-3-(2-methyl-5-(5-(2-(trifluoromethoxy)phenyl-1H-imidazol-2-yl)phenyl)-3,4-dihydropyrimido[4,5-d]pyrimidin-2(1H)-one (55.5 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 6-methylpyridine-3-amine (17.6 mg, 0.163 mmol) was used instead of 4-amino-1-methylpyrazole.

LCMS(ESI+, m/z) : 587.20 [M+H]+

¹H NMR (400 MHz, MeOD) δ 9.48 (d, *J* = 2.5 Hz, 1H), 8.49 (dd, *J* = 8.9, 2.6 Hz, 1H), 8.26 (s, 1H), 8.01 (d, *J* = 2.0 Hz, 1H), 7.92 (d, *J* = 8.0 Hz, 2H), 7.86 (s, 1H), 7.81 (d, *J* = 8.9 Hz, 1H), 7.66 (d, *J* = 8.1 Hz, 1H), 7.62 (dd, *J* = 7.3, 2.1 Hz, 1H), 7.59 - 7.52 (m, 2H), 4.91 (d, *J* = 14.3 Hz, 2H), 4.71 (d, *J* = 14.3 Hz, 2H), 3.50 (s, 3H), 2.73 (s, 3H), 2.37 (s, 3H).

### Example 10: 7-((1-(1-ethylpiperidin-4-yl)-1H-pyrazol-4-yl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

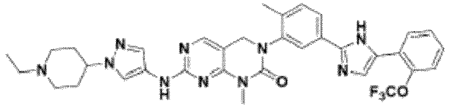

29.6 mg (41% yield) of the title compound was obtained by repeating the procedure of Step 1-10 of Example 1, except that, in Step 1-10 of Example 1, 7-chloro-1-methyl-3-(2-methyl-5-(5-(2-(trifluoromethoxy)phenyl-1H-imidazol-2-yl)phenyl)-3,4-dihydropyrimido[4,5-d]pyrimidin-2(1H)-one (55.5 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 1-(1-ethylpiperidin-4-yl)-1H-pyrazol-4-amine (31.6 mg, 0.163 mmol) was used instead of 4-amino-1-methylpyrazole.

LCMS (ESI+, m/z) : 673.30 [M+H]+

¹H NMR (400 MHz, MeOD) δ 7.97 (d, *J* = 1.0 Hz, 1H), 7.92 (s, 1H), 7.87 (d, *J* = 2.0 Hz, 1H), 7.82 (ddd, *J* = 12.5, 7.8, 1.9 Hz, 2H), 7.70 (s, 1H), 7.59 (s, 1H), 7.52 (d, *J* = 8.3 Hz, 1H), 7.50 - 7.40 (m, 3H), 4.76 (d, *J* = 13.9 Hz, 2H), 4.53 (d, *J* = 13.9 Hz, 2H), 4.48 - 4.37 (m, 1H), 3.65 (d, *J* = 12.6 Hz, 2H), 3.39 (s, 3H), 3.14 (t, *J* = 7.4 Hz, 2H), 3.12 - 3.01 (m, 2H), 2.30 (d, *J* = 13.9 Hz, 2H), 2.26 (s, 3H), 2.18 (d, *J* = 15.1 Hz, 2H), 1.29 (t, *J* = 7.4 Hz, 3H).

### Example 11: 7-(cyclopropylamine)-3-(5-(5-(2,3-difluorophenyl)-1H-imidazol-2-yl)-2-methylphenyl)-1-methyl-3,4-dihydropyrimido[4,5-d]pyrimidin-2(1H)-one

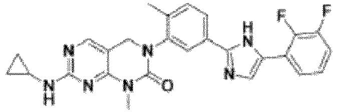

31.5 mg (60% yield) of the title compound was obtained by repeating the procedure of Step 1-10 of Example 1, except that, in Step 1-10 of Example 1, 7-chloro-3-(5-(5-(2, 3-difluoromethyl)-1H-imidazol-2-yl)-2-methylphenyl)-1-methyl-3,4-dihydropyrimido[4,5-d]pyrimidin-2(1H)-one (50.3 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and cyclopropylamine (11.3 µL, 0.163 mmol) was used instead of 4-amino-1-methylpyrazole.

LCMS (ESI+, m/z): 488.10 [M+H]+

¹H NMR (400 MHz, MeOD) δ 8.08 - 7.98 (m, 2H), 7.94 (tt, *J* = 3.5, 2.1 Hz, 2H), 7.73 (ddd, *J* = 9.5, 4.7, 1.9 Hz, 1H), 7.65 (t, *J* = 7.5 Hz, 1H), 7.47 - 7.28 (m, 2H), 4.82 (d, *J* = 14.7 Hz, 2H), 4.66 (d, *J* = 14.7 Hz, 2H), 3.52 (m, 1H), 3.47 (s, 3H), 2.37 (s, 3H), 1.00 (m, 2H), 0.82 - 0.69 (m, 2H).

### Example 12: 3-(5-(5-(2,3-difluorophenyl)-1H-imidazol-2-yl)-2-methylphenyl)-1-methyl-7-((6-methylpyridin-3-yl))amino)-3,4-dihydropyrimido[4,5-d]pyrimidin-2(1H)-one

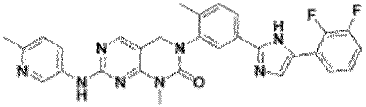

31.9 mg (55% yield) of the title compound was obtained by repeating the procedure of Step 1-10 of Example 1, except that, in Step 1-10 of Example 1, 7-chloro-3-(5-(5-(2, 3-difluoromethyl)-1H-imidazol-2-yl)-2-methylphenyl)-1-methyl-3,4-dihydropyrimido[4,5-d]pyrimidin-2(1H)-one (50.3 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 6-methylpyridine-3-amine (17.6 mg, 0.163 mmol) was used instead of 4-amino-1-methylpyrazole.

LCMS (ESI+, m/z) : 539.30 [M+H]+

¹H NMR (400 MHz, MeOD) δ 8.79 (d, *J* = 2.7 Hz, 1H), 8.16 (s, 1H), 8.13 (dd, *J* = 9.6, 2.7 Hz, 1H), 7.99 (d, *J* = 2.0 Hz, 1H), 7.91 (d, *J* = 6.5 Hz, 2H), 7.77 (dd, *J* = 7.7, 1.7 Hz, 1H), 7.66 (d, *J* = 8.1 Hz, 1H), 7.49 (ddd, *J* = 8.8, 7.4, 1.6 Hz, 1H), 7.33 (d, *J* = 9.7 Hz, 1H), 7.22 (dd, *J* = 8.4, 1.0 Hz, 1H), 7.13 (td, *J* = 7.6, 1.0 Hz, 1H), 4.90 (d, J = 14.1 Hz, 2H), 4.67 (d, *J* = 14.1 Hz, 2H), 4.00 (s, 3H), 3.79 (dd, *J* = 6.5, 3.8 Hz, 4H), 3.74 (dd, *J* = 7.1, 4.0 Hz, 2H), 3.67 (dd, *J* = 6.7, 4.2 Hz, 2H), 3.48 (s, 3H), 2.37 (s, 3H), 2.17 (s, 3H).

### Example 13: 1-methyl-3-(2-methyl-5-(5-(3-(trifluoromethyl)phenyl)-1H-imidazol-2-yl)phenyl)-7-((6-methylpyridin-3-yl)amino)-3,4-dihydropyrimido[4,5-d]pyrimidin-2(1H)-one

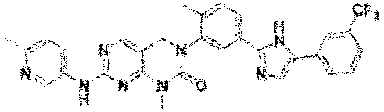

28.8 mg (47% yield) of the title compound was obtained by repeating the procedure of Step 1-10 of Example 1, except that, in Step 1-10 of Example 1, 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (53.8 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 6-methylpyridine-3-amine (17.6 mg, 0.163 mmol) was used instead of 4-amino-1-methylpyrazole.

LCMS (ESI+, m/z) : 571.20 [M+H]+

### Example 14: 7-((6-(4-acetylpiperazin-1-yl)pyridin-3-yl)amino)-1-methyl-3-(2-methyl-5-(5-(2-(trifloromethoxy)phenyl)-1H-imidazol-2-yl)phenyl)-3,4-dihydropyrimido[4,5-d]pyrimidin-2(1H)-one

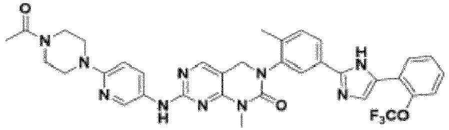

The title compound was obtained by repeating the procedure of Step 1-10 of Example 1, except that, in Step 1-10 of Example 1, 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (53.8 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 1-(4-(5-aminopyridin-2-yl)piperazin-1-yl)ethan-1-one (35.8 mg, 0.163 mmol) was used instead of 4-amino-1-methylpyrazole.

LCMS (ESI+, m/z) : 699.20 [M+H]+

### Example 15: 7-((6-(1-ethylpiperidin-4-yl)pyridin-3-yl)amino)-1-methyl-3-(2-methyl-5-(5-(2-(trifloromethoxy)phenyl)-1H-imidazol-2-yl)phenyl)-3,4-dihydropyrimido[4,5-d]pyrimidin-2(1H)-one

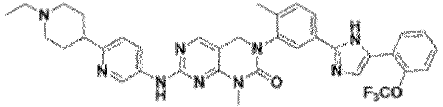

The title compound was obtained by repeating the procedure of Step 1-10 of Example 1, except that, in Step 1-10 of Example 1, 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (53.8 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 6-(1-ethylpiperidin-4-yl)pyridine-3-amine (33.4 mg, 0.163 mmol) was used instead of 4-amino-1-methylpyrazole.

LCMS (ESI+, m/z) : 684.20 [M+H]+

### Example 16: 7-((6-(4-ethylpiperazin-1-yl)pyridin-3-yl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

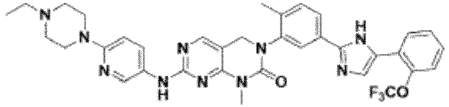

The title compound was obtained by repeating the procedure of Step 1-10 of Example 1, except that, in Step 1-10 of Example 1, 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (53.8 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 6-(4-ethylpiperazin-1-yl)pyridine-3-amine (33.6 mg, 0.163 mmol) was used instead of 4-amino-1-methylpyrazole.

LCMS (ESI+, m/z): 685.20 [M+H]+

### Example 17: 7-((1-(1-acetylpiperidin-4-yl)-1H-pyrazol-4-yl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

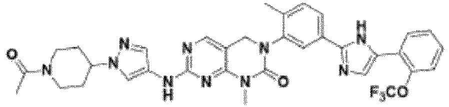

The title compound was obtained by repeating the procedure of Step 1-10 of Example 1, except that, in Step 1-10 of Example 1, 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (53.8 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 1-(4-(4-amino-1H-pyrazol-1-yl)piperidin-1-yl)ethan-1-one (33.9 mg, 0.163 mmol) was used instead of 4-amino-1-methylpyrazole.

LCMS (ESI+, m/z): 687.20 [M+H]+

### Example 18: 1-methyl-3-(2-methyl-5-(5-(2-(trifluoromethoxy)phenyl)-1H-imidazol-2-yl)phenyl)-7-((4-morpholinophenyl)amino)-3,4-dihydropyrimido[4,5-d]pyrimidin-2(1H)-one

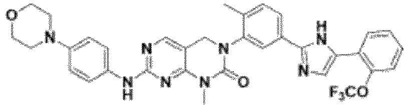

The title compound was obtained by repeating the procedure of Step 1-10 of Example 1, except that, in Step 1-10 of Example 1, 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (53.8 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 4-morpholinoaniline (29 mg, 0.163 mmol) was used instead of 4-amino-1-methylpyrazole.

LCMS (ESI+, m/z) : 657.20 [M+H]+

### Example 19: 1-methyl-3-(2-methyl-5-(5-(2-(trifluoromethoxy)phenyl)-1H-imidazol-2-yl)phenyl)-7-(phenylamino)-3,4-dihydropyrimido[4,5-d]pyrimidin-2(1H)-one

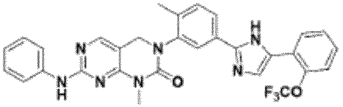

The title compound was obtained by repeating the procedure of Step 1-10 of Example 1, except that, in Step 1-10 of Example 1, 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (53.8 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and aniline (15.2 mg, 0.163 mmol) was used instead of 4-amino-1-methylpyrazole.

LCMS (ESI+, m/z) : 572.20 [M+H]+

### Example 20: 7-((4-methoxybenzyl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

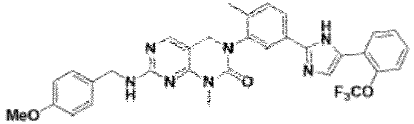

The title compound was obtained by repeating the procedure of Step 1-10 of Example 1, except that, in Step 1-10 of Example 1, 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (53.8 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and 4-methoxybenzylamine (22.3 mg, 0.163 mmol) was used instead of 4-amino-1-methylpyrazole.

LCMS (ESI+, m/z) : 616.15 [M+H]+

### Example 21: 7-(benzylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

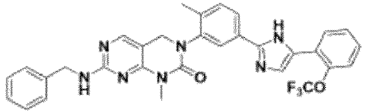

The title compound was obtained by repeating the procedure of Step 1-10 of Example 1, except that, in Step 1-10 of Example 1, 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (53.8 mg, 0.108 mmol) was used instead of 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one, and benzylamine (17.5 mg, 0.163 mmol) was used instead of 4-amino-1-methylpyrazole.

LCMS (ESI+, m/z): 586.15 [M+H]+

### Example 22: 7-(cyclopropylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

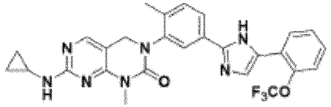

7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (500 mg, 0.897 mmol) was dissolved in 2-butanol (10 mL). Potassium carbonate (250 mg, 1.80 mmol) and cyclopropylamine (0.124 mL, 1.80 mmol) were added thereto, followed by stirring under reflux at 120°C for 18 hours. After completion of the reaction, the reaction solution was filtered through a celite pad, and the filtrate was concentrated using a vacuum distillation device. The resulting residue was purified by HPLC (high performance liquid chromatography/acetonitrile: water = 5: 95 → 95: 5 (v/v), 40 minutes), and the resulting solution was freeze-dried to obtain 465 mg (90% yield) of the title compound as a white solid.

LCMS (ESI+, m/z): 536.15 [M+H]+

### Example 23: 7-(cyclopentylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

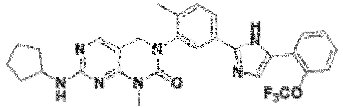

The title compound was obtained by repeating the procedure of Example 22, except that cyclopentylamine (18 µL, 0.18 mmol) was used instead of cyclopropylamine in Example 22.

LCMS (ESI+, m/z): 564.15 [M+H]+

### Example 24: 7-(cyclohexylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

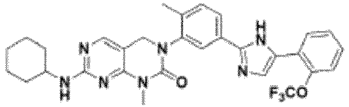

The title compound was obtained by repeating the procedure of Example 22, except that cyclohexylamine (21 µL, 0.18 mmol) was used instead of cyclopropylamine in Example 22.

LCMS (ESI+, m/z): 578.20 [M+H]+

### Example 25: 7-(butylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

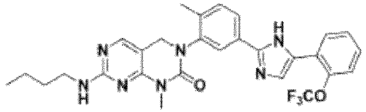

The title compound was obtained by repeating the procedure of Example 22, except that butylamine (18 µL, 0.18 mmol) was used instead of cyclopropylamine in Example 22.

LCMS (ESI+, m/z): 552.20 [M+H]+

### Example 26: 1-methyl-3-(2-methyl-5-(5-(2-(trifluoromethoxy)phenyl)-1H-imidazol-2-yl)phenyl)-7-(methylamino)-3,4-dihydropyrimido[4,5-d]pyrimidin-2(1H)-one

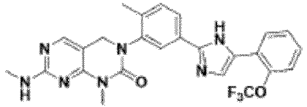

The title compound was obtained by repeating the procedure of Example 22, except that 2 M methylamine solution (in tetrahydrofuran, 90 µL, 0.18 mmol) was used instead of cyclopropylamine in Example 22.

LCMS (ESI+, m/z) : 510.15 [M+H]+

### Example 27: 7-amino-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one

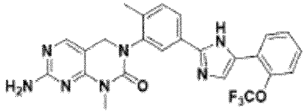

7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one (150 mg, 0.292 mmol) was dissolved in 2 M ammonia solution (in ethanol, 15 mL) and stirred under reflux at 120°C for 24 hours. After completion of the reaction, the reaction solution was concentrated using a vacuum distillation device. The resulting residue was purified by HPLC (high-performance liquid chromatography/acetonitrile: water = 5: 95 → 95: 5 (v/v), 40 minutes), and the resulting solution was freeze-dried to obtain 89 mg (61% yield) of the title compound as a white solid.

LCMS (ESI+, m/z) : 496.10 [M+H]+

### Example 28: 7-((6-(4-ethylpiperazin-1-yl)pyridin-3-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one

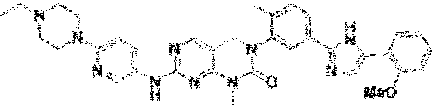

The title compound was obtained by repeating the procedure of Step 1-10 of Example 1, except that 6-(4-ethylpiperazin-1-yl)pyridine-3-amine (33.6 mg, 0.163 mmol) was used instead of 4-amino-1-methylpyrazole in Step 1-10 of Example 1.

LCMS (ESI+, m/z) : 631.30 [M+H]+

### Example 29: 7-((1-(1-acetylpiperidin-4-yl)-1H-pyrazol-4-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one

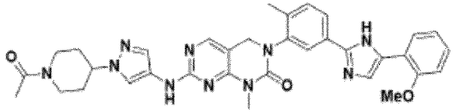

The title compound was obtained by repeating the procedure of Step 1-10 of Example 1, except that 1-(4-(4-amino-1H-pyrazol-1-yl)piperidin-1-yl)ethan-1-one (33.9 mg, 0.163 mmol) was used instead of 4-amino-1-methylpyrazole in Step 1-10 of Example 1.

LCMS (ESI+, m/z): 633.30 [M+H]+

### Example 30: 3-(5-(5-(2-methoxyphenyl)-1H-imidazol-2-yl)-2-methylphenyl)-1-methyl-7-(phenylamino)-3,4-dihydropyrimido[4,5-d]pyrimidin-2(1H)-one

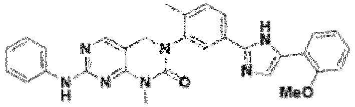

The title compound was obtained by repeating the procedure of Step 1-10 of Example 1, except that aniline (15.2 mg, 0.163 mmol) was used instead of 4-amino-1-methylpyrazole in Step 1-10 of Example 1.

LCMS (ESI+, m/z) : 518.20 [M+H]+

### Example 31: 7-(benzylamino)-3-(5-(5-(2-methoxyphenyl)-1H-imidazol-2-yl)-2-methylphenyl)-1-methyl-3,4-dihydropyrimido[4,5-d]pyrimidin-2(1H)-one

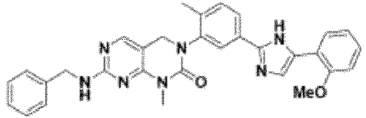

The title compound was obtained by repeating the procedure of Step 1-10 of Example 1, except that benzylamine (17.5 mg, 0.163 mmol) was used instead of 4-amino-1-methylpyrazole in Step 1-10 of Example 1.

LCMS (ESI+, m/z): 532.20 [M+H]+

### Example 32: 7-((4-methoxybenzyl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one

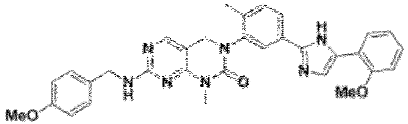

The title compound was obtained by repeating the procedure of Step 1-10 of Example 1, except that 4-methoxybenzylamine (22.3 mg, 0.163 mmol) was used instead of 4-amino-1-methylpyrazole in Step 1-10 of Example 1.

LCMS (ESI+, m/z) : 562.25 [M+H]+

### Example 33: 3-(5-(5-(2-methoxyphenyl)-1H-imidazol-2-yl)-2-methylphenyl)-1-methyl-7-(methylamino)-3,4-dihydropyrimido[4,5-d]pyrimidin-2(1H)-one

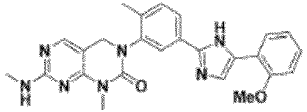

7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one (230 mg, 0.5 mmol) was dissolved in 2-butanol (7 mL). Potassium carbonate (139 mg, 1.0 mmol) and 2 M methylamine solution (in tetrahydrofuran, 0.50 mL, 1.00 mmol) were added thereto, followed by stirring under reflux at 120°C for 18 hours. After completion of the reaction, the reaction solution was filtered through a celite pad, and the filtrate was concentrated using a vacuum distillation device. The resulting residue was purified by HPLC (high-performance liquid chromatography/acetonitrile: water = 5: 95 → 95: 5 (v/v), 40 minutes), and the resulting solution was freeze-dried to obtain 109 mg (49% yield) of the title compound as a white solid.

LCMS (ESI+, m/z) : 456.15 [M+H]+

### Example 34: 7-(cyclopropylamino)-3-(5-(5-(2-methoxyphenyl)-1H-imidazol-2-yl)-2-methylphenyl)-1-methyl-3,4-dihydropyrimido[4,5-d]pyrimidin-2(1H)-one

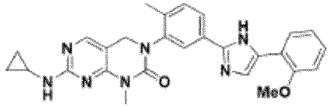

The title compound was obtained by repeating the procedure of Example 22, except that cyclopropylamine (0.069 mL, 1.0 mmol) was used instead of the 2 M methylamine solution in Example 33.

LCMS (ESI+, m/z): 482.20 [M+H]+

### Example 35: 7-(butylamino)-3-(5-(5-(2-methoxyphenyl)-1H-imidazol-2-yl)-2-methylphenyl)-1-methyl-3,4-dihydropyrimido[4,5-d]pyrimidin-2(1H)-one

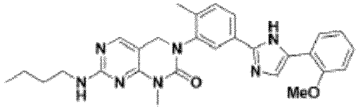

The title compound was obtained by repeating the procedure of Example 22, except that butylamine (100 mL, 1.0 mmol) was used instead of the 2 M methylamine solution in Example 33.

LCMS (ESI+, m/z) : 498.25 [M+H]+

### Example 36: 7-(cyclopentylamino)-3-(5-(5-(2-methoxyphenyl)-1H-imidazol-2-yl)-2-methylphenyl)-1-methyl-3,4-dihydropyrimido[4,5-d]pyrimidin-2(1H)-one

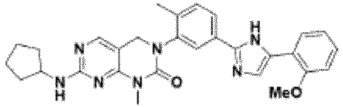

The title compound was obtained by repeating the procedure of Example 22, except that cyclopentylamine (100 mL, 1.0 mmol) was used instead of the 2 M methylamine solution in Example 33.

LCMS (ESI+, m/z): 510.25 [M+H]+

### Example 37: 7-(cyclohexylamino)-3-(5-(5-(2-methoxyphenyl)-1H-imidazol-2-yl)-2-methylphenyl)-1-methyl-3,4-dihydropyrimido[4,5-d]pyrimidin-2(1H)-one

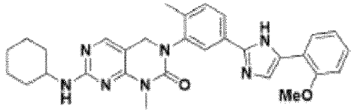

The title compound was obtained by repeating the procedure of Example 22, except that cyclohexylamine (117 mL, 0.18 mmol) was used instead of the 2 M methylamine solution in Example 33.

LCMS (ESI+, m/z) : 510.25 [M+H]+

### Example 38: 7-amino-3-(5-(5-(2-methoxyphenyl)-1H-imidazol-2-yl)-2-methylphenyl)-1-methyl-3,4-dihydropyrimido[4,5-d]pyrimidin-2(1H)-one

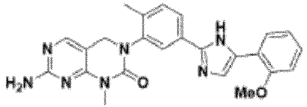

The title compound was obtained by repeating the procedure of Example 27, except that 7-chloro-3-(5-(5-(2-methoxyphenyl)-1H-imidazol-2-yl)-2-methylphenyl)-1-methyl-3,4-dihydropyrimido[4,5-d]pyrimidin-2(1H)-one produced in Step 1-9 of Example 1 was used instead of 7-chloro-1-methyl-3-(2-methyl-5-(5-(3-(trifluoromethyl)phenyl)-1H-imidazol-2-yl)phenyl)-3,4-dihydropyrimido[4,5-d]pyrimidin-2(1H)-one in Example 27.

LCMS(ESI+, m/z): 442.10 [M+H]+

### [Representative Synthesis Example 2]

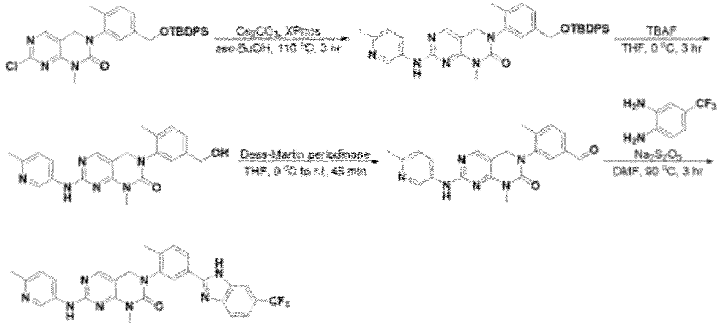

### Example 39: 1-methyl-3-(2-methyl-5-(6-(trifluoromethyl)-1H-benzo[d]imidazol-2-yl)phenyl)-7-((6-methylpyridin-3-yl)amino)-3,4-dihydropyrimido[4,5-d]pyrimidin-2(1H)-one

### Step 2-1) 3-(5-(((tert-butyldiphenylsilyl)oxy)methyl)-2-methylphenyl)-1-methyl-7-((6-methylpyridin-3-yl)amino)-3,4-dihydropyrimido[4,5-d]pyrimidin-2(1H)-one

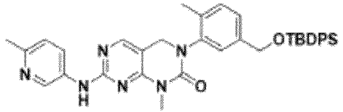

3-(5-(((tert-butyldiphenylsilyl)oxy)methyl)-2-methylphenyl)-7-chloro-1-methyl-3,4-dihydropyrimido[4,5-d]pyrimidin-2(1H)-one (500 mg, 0.89 mmol) produced in Step 1-5 of Example 1 was dissolved in 2-butanol (10 mL). Cesium carbonate (584 mg, 1.80 mmol), 6-methylpyridine-3-amine (194 mg, 1.80 mmol), XPhos (86 mg, 0.18 mmol) and Pd₂(dba)₃ (164 mg, 0.18 mmol) were added thereto, followed by stirring under reflux at 110°C for 3 hours. After completion of the reaction, the mixture solution was filtered through a celite pad, and the filtrate was concentrated using a vacuum distillation device. The resulting residue was purified by MPLC (dichloromethane: methanol = 1: 19 (v/v)), and the resulting solution was concentrated under reduced pressure to obtain 210 mg (38% yield) of the title compound as a white solid.

LCMS (ESI+, m/z) : 629.30 [M+H]+

¹H NMR (400 MHz, CDCl₃) δ 8.68 (d, *J* = 2.6 Hz, 1H), 8.00 (dd, *J* = 8.4, 2.7 Hz, 1H), 7.97 (s, 1H), 7.68 (ddd, *J* = 8.0, 3.3, 1.6 Hz, 5H), 7.40 (dddd, *J* = 18.0, 10.3, 5.6, 1.8 Hz, 7H), 7.18 - 7.09 (m, 2H), 4.75 (s, 2H), 4.63 (d, *J* = 14.0 Hz, 1H), 4.42 (d, *J* = 14.0 Hz, 1H), 3.44 (s, 3H), 2.53 (s, 3H), 2.22 (s, 3H), 1.09 (s, 9H).

### Step 2-2) 3-(5-(hydroxymethyl)-2-methylphenyl)-1-methyl-7-((6-methylpyridin-3-yl)amino)-3,4-dihydropyrimido[4,5-d]pyrimidin-2(1H)-one

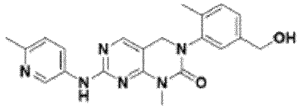

3-(5-(((tert-butyldiphenylsilyl)oxy)methyl)-2-methylphenyl)-1-methyl-7-((6-methylpyridin-3-yl)amino)-3,4-dihydropyrimido[4,5-d]pyrimidin-2(1H)-one (140 mg, 0.22 mmol) produced in Step 2-1 of Example 39 was dissolved in anhydrous dry tetrahydrofuran (14.0 mL), and the reaction vessel was placed and cooled in an ice water bath at 0°C. 1 M tetrabutylammonium fluoride (tetrahydrofuran solution, 0.28 mL, 0.28 mmol) was added thereto dropwise, followed by stirring at 0°C for 3 hours. After completion of the reaction, a saturated aqueous solution of ammonium chloride was added dropwise to the mixture solution, followed by warming to room temperature and extraction with ethyl acetate. The combined organic layers were dried with anhydrous sodium sulfate and concentrated. The resulting residue was purified by MPLC (dichloromethane: methanol = 1: 9 (v/v)), and the resulting solution was concentrated under reduced pressure to obtain 63 mg (73%) of the title compound as a white solid.

LCMS (ESI+, m/z): 391.20 [M+H]+

¹H NMR (400 MHz, CDCl₃) δ 8.66 (d, *J* = 3.7 Hz, 2H), 8.01 (dd, *J* = 8.4, 2.7 Hz, 1H), 7.97 (s, 1H), 7.29 (d, *J* = 6.7 Hz, 2H), 7.21 - 7.11 (m, 2H), 4.67 (d, *J* = 15.2 Hz, 3H), 4.47 (d, *J* = 14.0 Hz, 1H), 3.45 (s, 3H), 2.54 (s, 3H), 2.24 (s, 3H).

### Step 2-3) 4-methyl-3-(1-methyl-7-((6-methylpyridin-3-yl)amino)-2-oxo-1,4-dihydropyrimido[4,5-d]pyrimidin-3(2H)-yl)benzaldehyde

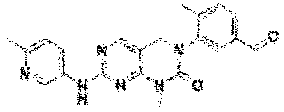

3-(5-(hydroxymethyl)-2-methylphenyl)-1-methyl-7-((6-methylpyridin-3-yl)amino)-3,4-dihydropyrimido[4,5-d]pyrimidin-2(1H)-one (100 mg, 0.256 mmol) produced in Step 2-2 of Example 39 was dissolved in anhydrous dry tetrahydrofuran (6.0 mL) and then cooled to 0 °C. Thereafter, Dess-Martin Periodinane (163 mg, 0.384 mmol) was added thereto, followed by stirring at room temperature for 45 minutes. After completion of the reaction, a saturated aqueous solution of sodium hydrogen carbonate was added dropwise to the mixture solution, followed by extraction with dichloromethane. The combined organic layers were washed with brine, dried with anhydrous sodium sulfate, and then concentrated. The resulting residue was purified by MPLC (dichloromethane: methanol = 1: 30 (v/v)), and the resulting solution was concentrated under reduced pressure to obtain 89 mg (89% yield) of the title compound as a white solid.

LCMS (ESI+, m/z) : 389.15 [M+H]+

### Step 2-4) 1-methyl-3-(2-methyl-5-(6-(trifluoromethyl)-1H-benzo[d]imidazol-2-yl)phenyl)-7-((6-methylpyridin)-3-yl)amino)-3,4-dihydropyrimido[4,5-d]pyrimidin-2(1H)-one

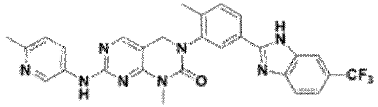

4-methyl-3-(1-methyl-7-((6-methylpyridin-3-yl)amino)-2-oxo-1,4-dihydropyrimido[4,5-d]pyrimidin-3(2H)-yl)benzaldehyde (30 mg, 0.077 mmol) produced in Step 2-3 of Example 39 was dissolved in N,N-dimethylformamide (1.0 mL). 4-(trifluoromethyl)benzene-1,2-diamine (14.9 mg, 0.085 mmol) and sodium metabisulfite (16.2 mg, 0.085 mmol) were added thereto, followed by stirring under reflux at 90°C for 3 hours. After completion of the reaction, water was added to the mixture solution, followed by extraction with dichloromethane. The combined organic layers were dried with anhydrous sodium sulfate and then concentrated. The resulting residue was purified by HPLC (acetonitrile: water = 5: 95 → 95:5 (v/v), 40 minutes), and the resulting solution was freeze-dried to obtain 12.2 mg (29% yield) of the title compound as a white solid.

LCMS (ESI+, m/z) : 545.20 [M+H]+

¹H NMR (400 MHz, MeOD) δ 9.49 (d, *J* = 2.5 Hz, 1H), 8.48 (dd, *J* = 8.7, 2.5 Hz, 1H), 8.25 (s, 1H), 8.14 (s, 1H), 8.08 - 8.01 (m, 1H), 7.97 (s, 1H), 7.85 - 7.78 (m, 2H), 7.64 (d, *J* = 8.6 Hz, 1H), 7.60 (d, *J* = 8.2 Hz, 1H), 4.93 (d, *J* = 14.3 Hz, 1H), 4.70 (d, *J* = 14.3 Hz, 1H), 3.49 (s, 3H), 2.72 (s, 3H), 2.36 (s, 3H).

### Example 40: 3-(5-(5,6-difluoro-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one

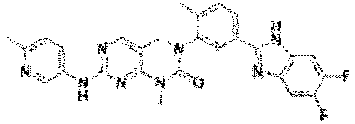

The title compound was obtained by repeating the procedure of Step 2-4 of Example 39, except that 4,5-difluorobenzene-1,2-diamine (12.2 mg, 0.085 mmol) was used instead of 4-(trifluoromethyl)benzene-1,2-diamine in Step 2-4 of Example 39.

LCMS (ESI+, m/z): 513.20 [M+H]+

### Example 41: 3-(5-(5,6-dibromo-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one

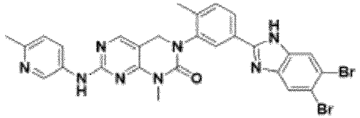

The title compound was obtained by repeating the procedure of Step 2-4 of Example 39, except that 4,5-dibromobenzene-1,2-diamine (22.4 mg, 0.085 mmol) was used instead of 4-(trifluoromethyl)benzene-1,2-diamine in Step 2-4 of Example 39.

LCMS (ESI+, m/z) : 633.00 [M+H]+

### Example 42: 1-methyl-3-(2-methyl-5-(7-methyl-1H-benzo[d]imidazol-2-yl)phenyl)-7-((6-methylpyridin-3-yl)amino)-3,4-dihydropyrimido[4,5-d]pyrimidin-2(1H)-one

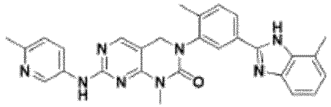

The title compound was obtained by repeating the procedure of Step 2-4 of Example 39, except that 3-methylbenzene-1,2-diamine (10.4 mg, 0.085 mmol) was used instead of 4-(trifluoromethyl)benzene-1,2-diamine in Step 2-4 of Example 39.

LCMS(ESI+, m/z): 491.25 [M+H]+

### Example 43: 3-(5-(6-bromo-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one

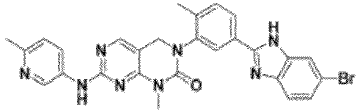

The title compound was obtained by repeating the procedure of Step 2-4 of Example 39, except that 4-bromobenzene-1,2-diamine (15.8 mg, 0.085 mmol) was used instead of 4-(trifluoromethyl)benzene-1,2-diamine in Step 2-4 of Example 39.

LCMS (ESI+, m/z) : 555.10 [M+H]+

### Example 44: 3-(5-(6-chloro-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one

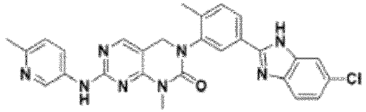

The title compound was obtained by repeating the procedure of Step 2-4 of Example 39, except that 4-chlorobenzene-1,2-diamine (12.1 mg, 0.085 mmol) was used instead of 4-(trifluoromethyl)benzene-1,2-diamine in Step 2-4 of Example 39.

LCMS (ESI+, m/z): 511.20 [M+H]+

### Example 45: 1-methyl-3-(2-methyl-5-(6-methyl-1H-benzo[d]imidazol-2-yl)phenyl)-7-((6-methylpyridin-3-yl)amino)-3,4-dihydropyrimido[4,5-d]pyrimidin-2(1H)-one

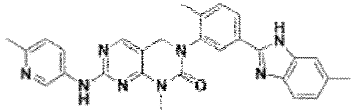

The title compound was obtained by repeating the procedure of Step 2-4 of Example 39, except that 4-methylbenzene-1,2-diamine (10.4 mg, 0.085 mmol) was used instead of 4-(trifluoromethyl)benzene-1,2-diamine in Step 2-4 of Example 39.

LCMS (ESI+, m/z) : 491.25 [M+H]+

### Example 46: 3-(5-(5,6-dichloro-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one

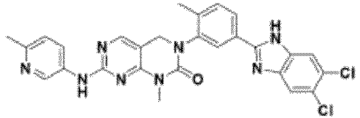

The title compound was obtained by repeating the procedure of Step 2-4 of Example 39, except that 4,5-dichlorobenzene-1,2-diamine (15.0 mg, 0.085 mmol) was used instead of 4-(trifluoromethyl)benzene-1,2-diamine in Step 2-4 of Example 39.

LCMS (ESI+, m/z) : 491.25 [M+H]+

### Example 47: 3-(5-(5,6-dimethyl-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one

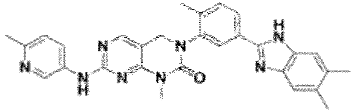

The title compound was obtained by repeating the procedure of Step 2-4 of Example 39, except that 4,5-dimethylbenzene-1,2-diamine (11.6 mg, 0.085 mmol) was used instead of 4-(trifluoromethyl)benzene-1,2-diamine in Step 2-4 of Example 39.

LCMS (ESI+, m/z): 505.25 [M+H]+

### Example 48: 1-methyl-3-(2-methyl-5-(6-(trifluoromethyl)-1H-benzo[d]imidazol-2-yl)phenyl)-7-(phenylamino)-3,4-dihydropyrimido[4,5-d]pyrimidin-2(1H)-one

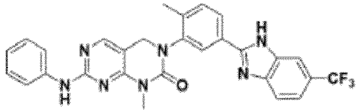

The title compound was obtained by repeating the procedure of Step 2-4 of Example 39, except that 4-methyl-3-(1-methyl-2-oxo-7-(phenylamino)-1,4-dihydropyrimido[4,5-d]pyrimidin-3(2H)-yl)benzaldehyde (30 mg, 0.080 mmol) was used instead of 4-methyl-3-(1-methyl-7-((6-methylpyridin-3-yl)amino)-2-oxo-1,4-dihydropyrimido[4,5-d]pyrimidin-3(2H)-yl)benzaldehyde in Step 2-4 of Example 39.

LCMS (ESI+, m/z) : 530.20 [M+H]+

### Example 49: 1-methyl-3-(2-methyl-5-(6-(trifluoromethyl)-1H-benzo[d]imidazol-2-yl)phenyl)-7-(phenylamino)-3,4-dihydropyrimido[4,5-d]pyrimidin-2(1H)-one

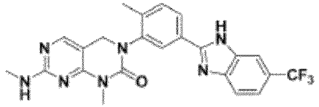

The title compound was obtained by repeating the procedure of Step 2-4 of Example 39, except that 4-methyl-3-(1-methyl-7-(methylamino)-2-oxo-1,4-dihydropyrimido[4,5-d]pyrimidin-3(2H)-yl)benzaldehyde (30 mg, 0.096 mmol) was used instead of 4-methyl-3-(1-methyl-7-((6-methylpyridin-3-yl)amino)-2-oxo-1,4-dihydropyrimido[4,5-d]pyrimidin-3(2H)-yl)benzaldehyde in Step 2-4 of Example 39.

LCMS (ESI+, m/z): 468.20 [M+H]+

### Example 50: 7-(cyclopropylamino)-1-methyl-3-(2-methyl-5-(6-(trifluoromethyl)-1H-benzo[d]imidazol-2-yl)phenyl)-3,4-dihydropyrimido[4,5-d]pyrimidin-2(1H)-one

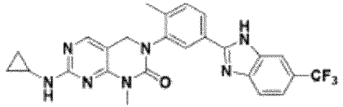

The title compound was obtained by repeating the procedure of Step 2-4 of Example 39, except that 3-(7-(cyclopropylamino)-1-methyl-2-oxo-1,4-dihydropyrimido[4,5-d]pyrimidin-3(2H)-yl)-4-methylbenzaldehyde (30 mg, 0.089 mmol) was used instead of 4-methyl-3-(1-methyl-7-((6-methylpyridine-3-yl)amino)-2-oxo-1,4-dihydropyrimido[4,5-d]pyrimidin-3(2H)-yl)benzaldehyde in Step 2-4 of Example 39.

LCMS (ESI+, m/z): 494.20 [M+H]+

### Example 51: 7-((2,6-dimethylpyridin-3-yl)amino)-1-methyl-3-(2-methyl-5-(6-(trifluoromethyl)-1H-benzo[d]imidazol-2-yl)phenyl)-3,4-dihydropyrimido[4,5-d]pyrimidin-2(1H)-one

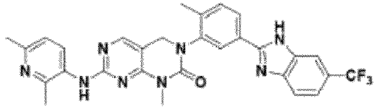

The title compound was obtained by repeating the procedure of Step 2-4 of Example 39, except that 3-(7-((2,6-dimethylpyridin-3-yl)amino)-1-methyl-2-oxo-1,4-dihydropyrimido[4,5-d]pyrimidin-3(2H)-yl)-4-methylbenzaldehyde (30 mg, 0.075 mmol) was used instead of 4-methyl-3-(1-methyl-7-((6-methylpyridin-3-yl)amino)-2-oxo-1,4-dihydropyrimido[4,5-d]pyrimidin-3(2H)-yl)benzaldehyde in Step 2-4 of Example 39.

LCMS (ESI+, m/z) : 559.20 [M+H]+

### [Representative Synthesis Example 3]

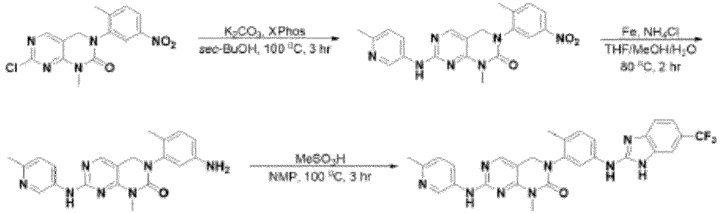

### Example 52: 1-methyl-3-(2-methyl-5-(6-(trifluoromethyl)-1H-benzo[d]imidazol-2-yl)phenyl)-7-((6-methylpyridin-3-yl)amino)-3,4-dihydropyrimido[4,5-d]pyrimidin-2(1H)-one

### Step 3-1) 1-methyl-3-(2-methyl-5-nitrophenyl)-7-((6-methylpyridin-3-yl)amino)-3,4-dihydropyrimido[4,5-d]pyrimidin-2(1H)-one

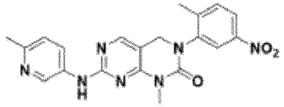

7-chloro-1-methyl-3-(2-methyl-5-nitrophenyl)-3,4-dihydropyrimido[4,5-d]pyrimidin-2(1H)-one (5.0 g, 15.01 mmol) was dissolved in 2-butanol (55 mL). Potassium carbonate (4.14 g, 29.96 mmol), 6-methylpyridine-3-amine (969 mg, 8.97 mmol), XPhos (853 mg, 1.79 mmol) and Pd₂(dba)₃ (1.64 g, 1.79 mmol) were added thereto, followed by stirring under reflux at 110°C for 3 hours. After completion of the reaction, the mixture solution was filtered through a celite pad, and the filtrate was concentrated using a vacuum distillation device. The resulting residue was purified by a high-performance automatic separation and purification system (dichloromethane: methanol = 1: 19 (v/v)), and the resulting solution was concentrated under reduced pressure to obtain 4.8 g (79% yield) of the title compound as a yellow solid.

LCMS (ESI+, m/z): 405.20 [M+H]+

¹H NMR (400 MHz, DMSO-d₆) δ 9.65 (s, 1H), 8.78 (d, J = 2.4 Hz, 1H), 8.34 (d, J = 2.4 Hz), 8.15-8.12 (m, 2H), 8.05 (dd, J = 8.3 Hz, J = 2.8 Hz, 1H), 7.62 (d, J = 8.3 Hz, 1H), 7.19 (d, J = 8.3 Hz, 1H), 4.89 (d, J = 13.5 Hz, 1H), 4.50 (d, J = 13.5 Hz, 1H), 3.34 (s, 3H), 2.40 (s, 3H), 2.29 (s,3H).

### Step 3-2) 3-(5-amino-2-methylphenyl)-1-methyl-7-((6-methylpyridin-3-yl)amino)-3,4-dihydropyrimido[4,5-d]pyrimidin-2(1H)-one

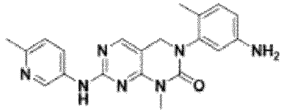

1-methyl-3-(2-methyl-5-nitrophenyl)-7-((6-methylpyridin-3-yl)amino)-3,4-dihydropyrimido[4,5-d]pyrimidin-2(1H)-one (3.5 g, 8.64 mmol) produced in Step 3-1 of Example 52 was dissolved in tetrahydrofuran (120 mL), and methanol (60 mL) and water (30 mL) were added dropwise thereto. Iron (4.83 g, 86.4 mmol) and ammonium chloride (9.24 g, 172.8 mmol) were added thereto at room temperature, followed by stirring under reflux at 80°C for 2 hours. After completion of the reaction, the reaction solution was cooled to room temperature and filtered through a celite pad. The filtrate was neutralized by dropwise addition of a saturated aqueous solution of sodium hydrogen carbonate and extracted with dichloromethane. The combined organic layers were dried with anhydrous sodium sulfate and then concentrated to 3.19 g (98% yield) of the title compound as a yellow solid.

LCMS (ESI+, m/z) : 376.20 [M+H]+

¹H NMR (400 MHz, DMSO-d₆) δ 9.62 (s, 1H), 8.79 (s, 1H), 8.12 (s, 1H), 8.04 (dd, J = 2.8 Hz, J = 8.4 Hz, 1H), 7.16 (d, J = 8.4 Hz, 1H), 6.94 (d, J = 8.8 Hz, 1H), 6.47 (m, 2H), 4.97 (s, 2H), 4.59 (d, J = 14.4 Hz, 1H), 4.43 (d, J = 14.4 Hz, 1H), 3.31 (s, 3H), 2.40 (s, 3H), 1.95 (s, 3H)

### Step 3-3) 5-(trifluoromethyl)-1,3-dihydro-2H-benzo[d]imidazol-2-one

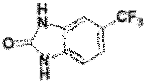

4-(trifluoromethyl)benzene-1,2-diamine (350 mg, 1.99 mmol) was dissolved in anhydrous dry tetrahydrofuran (8.0 mL). 1,1-carbonyldiimidazole (420 mg, 2.59 mmol) was added thereto, followed by stirring at room temperature for 12 hours. After completion of the reaction, a saturated aqueous solution of ammonium chloride was added dropwise to the mixture solution, followed by extraction with ethyl acetate. The combined organic layers were washed with brine, dried with anhydrous sodium sulfate, and then concentrated. The resulting residue was purified by MPLC (ethyl acetate: hexane = 1: 2(v/v)), and the resulting solution was concentrated under reduced pressure to obtain 338 mg (84% yield) of the title compound.

LCMS (ESI+, m/z): 203.05 [M+H]+

¹H NMR (400 MHz, DMSO-d₆) δ 11.03 (2H, s), 7.28 (d, J = 8.0 Hz, 1H), 7.16 (1 H, s), 7.09 (d, J = 8.3 Hz, 1H)

### Step 3-4) 2-chloro-5-(trifluoromethyl)-1H-benzo[d]imidazole

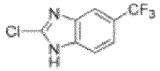

5-(trifluoromethyl)-1,3-dihydro-2H-benzo[d]imidazol-2-one (300 mg, 1.48 mmol) produced in Step 3-3 of Example 52 was added to phosphorus oxychloride (7.0 mL) and stirred under reflux at 115°C for 2 hours. The reaction solution was cooled to room temperature and then concentrated using a vacuum distillation device. The concentrate was dissolved in ethyl acetate and recrystallized at room temperature to obtain 279 mg (86% yield) of the title compound.

LCMS (ESI+, m/z) : 221.0 [M+H]+

¹H NMR (400 MHz, DMSO-d6) δ 7.87 (1H, s), 7.69 (1H, d), 7.57 (1H, dd)

### Step 3-5) 1-methyl-3-(2-methyl-5-(6-(trifluoromethyl)-1H-benzo[d]imidazol-2-yl)phenyl)-7-((6-methylpyridin)-3-yl)amino)-3,4-dihydropyrimido[4,5-d]pyrimidin-2(1H)-one

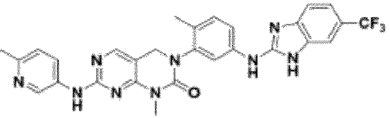

3-(5-amino-2-methylphenyl)-1-methyl-7-((6-methylpyridin-3-yl)amino)-3,4-dihydropyrimido[4,5-d]pyrimidin-2(1H)-one (65 mg, 0.173 mmol) produced in Step 3-2 of Example 52 was dissolved in N-methylpyrrolidinone (3.4 mL). 2-chloro-5-(trifluoromethyl)-1H-benzo[d]imidazole (76 mg, 0.346 mmol) produced in Step 3-4 of Example 15 and methanesulfonic acid (3.4 µL, 0.052 mmol) were added thereto, followed by stirring under reflux at 100°C for 3 hours. After completion of the reaction, the reaction solution was concentrated using a vacuum distillation device, and then the resulting residue was purified by HPLC (high-performance liquid chromatography/acetonitrile: water = 5 : 95 → 95: 5 (v/v), 40 minutes), and the resulting solution was freeze-dried to obtain 58.3 mg (60% yield) of the title compound as a white solid.

LCMS (ESI+, m/z) : 560.20 [M+H]+

¹H NMR (400 MHz, MeOD) δ 9.48 (d, *J* = 2.5 Hz, 1H), 8.48 (dd, *J* = 8.9, 2.5 Hz, 1H), 8.24 (s, 1H), 7.81 (d, *J* = 8.9 Hz, 1H), 7.70 (s, 1H), 7.66 - 7.52 (m, 4H), 7.43 (dd, *J* = 8.2, 2.3 Hz, 1H), 4.83 (d, *J* = 14.3 Hz, 1H), 4.66 (d, *J* = 14.3 Hz, 1H), 3.49 (s, 3H), 2.73 (s, 3H), 2.32 (s, 3H).

### Example 53: 3-(5-((5,6-difluoro-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one

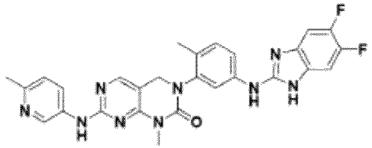

39.3 mg (43% yield) of the title compound was obtained by repeating the procedure of Step 3-5 of Example 52, except that 2-chloro-5,6-diflioro-1H-benzo[d]imidazole (65 mg, 0.346 mmol) was used instead of 2-chloro-5-(trifluoromethyl)-1H-benzo[d]imidazole in Step 3-5 of Example 52.

LCMS (ESI+, m/z): 528.20 [M+H]+

¹H NMR (400 MHz, MeOD) δ 9.48 (d, J = 2.5 Hz, 1H), 8.49 (dd, J = 8.9, 2.6 Hz, 1H), 8.24 (s, 1H), 7.81 (d, J = 8.9 Hz, 1H), 7.54 (d, J = 8.2 Hz, 1H), 7.50 (d, J = 2.2 Hz, 1H), 7.38 (dt, J = 13.1, 5.2 Hz, 3H), 6.96 (t, J = 8.6 Hz, 1H), 4.82 (d, J = 14.3 Hz, 1H), 4.66 (d, J = 14.3 Hz, 1H), 3.49 (s, 3H), 2.73 (s, 3H), 2.31 (s, 3H).

### Example 54: 3-(5-((1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one

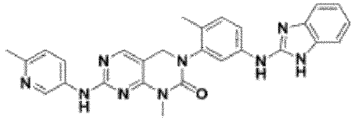

60.3 mg (71% yield) of the title compound was obtained by repeating the procedure of Step 3-5 of Example 52, except that 2-chloro-1H-benzo[d]imidazole (53 mg, 0.346 mmol) was used instead of 2-chloro-5-(trifluoromethyl)-1H-benzo[d]imidazole in Step 3-5 of Example 52.

LCMS (ESI+, m/z): 492.22 [M+H]+

### Example 55: 3-(5-((6-bromo-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one

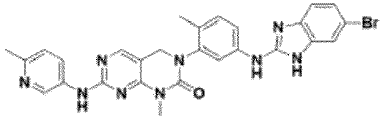

The title compound was obtained by repeating the procedure of Step 3-5 of Example 52, except that 6-bromo-2-chloro-1H-benzo[d]imidazole (79.2 mg, 0.346 mmol) was used instead of 2-chloro-5-(trifluoromethyl)-1H-benzo[d]imidazole in Step 3-5 of Example 52.

LCMS (ESI+, m/z): 570.15 [M+H]+

### Example 56: 3-(5-((6-chloro-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one

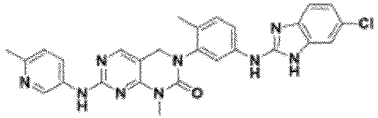

The title compound was obtained by repeating the procedure of Step 3-5 of Example 52, except that 2,6-dichloro-1H-benzo[d]imidazole (64.3 mg, 0.346 mmol) was used instead of 2-chloro-5-(trifluoromethyl)-1H-benzo[d]imidazole in Step 3-5 of Example 52.

LCMS (ESI+, m/z) : 526.20 [M+H]+

### Example 57: 1-methyl-3-(2-methyl-5-((6-methyl-1H-benzo[d]imidazol-2-yl)amino)phenyl)-7-((6-methylpyridin-3-yl)amino)-3,4-dihydropyrimido[4,5-d]pyrimidin-2(1H)-one

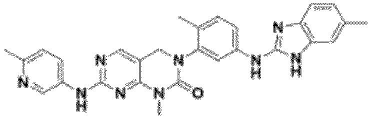

The title compound was obtained by repeating the procedure of Step 3-5 of Example 52, except that 2-chloro-6-methyl-1H-benzo[d]imidazole (57.4 mg, 0.346 mmol) was used instead of 2-chloro-5-(trifluoromethyl)-1H-benzo[d]imidazole in Step 3-5 of Example 52.

LCMS (ESI+, m/z): 506.25 [M+H]+

### Example 58: 3-(5-((6-dibromo-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one

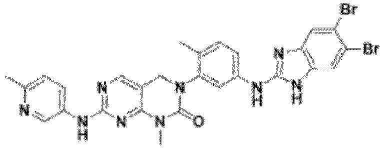

The title compound was obtained by repeating the procedure of Step 3-5 of Example 52, except that 5,6-dibromo-2-chloro-1H-benzo[d]imidazole (106.5 mg, 0.346 mmol) was used instead of 2-chloro-5-(trifluoromethyl)-1H-benzo[d]imidazole in Step 3-5 of Example 52.

LCMS (ESI+, m/z): 648.05 [M+H]+

### Example 59: 3-(5-((6-dichloro-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one

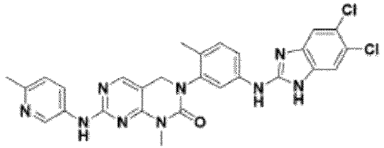

The title compound was obtained by repeating the procedure of Step 3-5 of Example 52, except that 2,5,6-trichloro-1H-benzo[d]imidazole (76.1 mg, 0.346 mmol) was used instead of 2-chloro-5-(trifluoromethyl)-1H-benzo[d]imidazole in Step 3-5 of Example 52.

LCMS(ESI+, m/z) : 560.15 [M+H]+

### Example 60: 3-(5-((5,6-dimethyl-1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one

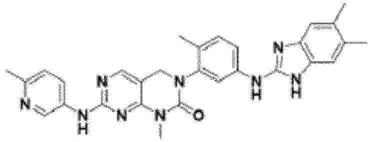

The title compound was obtained by repeating the procedure of Step 3-5 of Example 52, except that 2-chloro-5,6-dimethyl-1H-benzo[d]imidazole (62.3 mg, 0.346 mmol) was used instead of 2-chloro-5-(trifluoromethyl)-1H-benzo[d]imidazole in Step 3-5 of Example 52.

LCMS (ESI+, m/z): 520.25 [M+H]+

### Example 61: 3-(5-((5,6-difluoro-1H-benzo[d]imidazol-2-yl)amino)-2-methylphenyl)-1-methyl-7-(phenylamino)-3,4-dihydropyrimido[4,5-d]pyrimidin-2(1H)-one

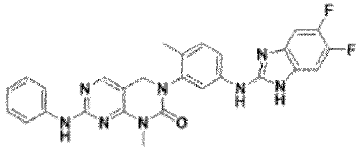

The title compound was obtained by repeating the procedure of Step 3-5 of Example 52, except that 3-(5-amino-2-methylphenyl)-1-methyl-7-(phenylamino)-3,4-**dihydropyrimido[4,5-d]pyrimidin-2(1H)-one** (62.3 mg, 0.173 mmol) was used instead of 3-(5-amino-2-methylphenyl)-1-methyl-7-((6-methylpyridin-3-yl)amino)-3,4-dihydropyrimido[4,5-d]pyrimidin-2(1H)-one produced in Step 3-2 of Example 52, and 2-chloro-5,6-difluoro-1H-benzo[d]imidazole (65 mg, 0.346 mmol) was used instead of 2-chloro-5-(trifluoromethyl)-1H-benzo[d]imidazole produced in Step 3-4 of Example 52.

LCMS (ESI+, m/z) : 520.20 [M+H]+

### Example 62: 3-(5-((5,6-difluoro-1H-benzo[d]imidazol-2-yl)amino)-2-methylphenyl)-7-((2,6-dimethylpyridin-3-yl)amino)-1-methyl-3,4-dihydropyrimido[4,5-d]pyrimidin-2(1H)-one

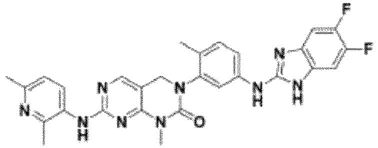

The title compound was obtained by repeating the procedure of Step 3-5 of Example 52, except that 3-(5-amino-2-methylphenyl)-7-((2,6-dimethylpyridin-3-yl)amino)-1-methyl-3,4-dihydropyrimido[4,5-d]pyrimidin-2(1H)-one (67.3 mg, 0.173 mmol) was used instead of 3-(5-amino-2-methylphenyl)-1-methyl-7-((6-methylpyridin-3-yl)amino)-3,4-**dihydropyrimido[4,5-d]pyrimidin-2(1H)-one** produced in Step 3-2 of Example 52, and 2-chloro-5,6-difluoro-1H-benzo[d]imidazole (65 mg, 0.346 mmol) was used instead of 2-chloro-5-(trifluoromethyl)-1H-benzo[d]imidazole produced in Step 3-4 of Example 52.

LCMS (ESI+, m/z): 542.25 [M+H]+

### Example 63: 3-(5-((5,6-difluoro-1H-benzo[d]imidazol-2-yl)amino)-2-methylphenyl)-1-methyl-7-(methylamino)-3,4-dihydropyrimido[4,5-d]pyrimidin-2(1H)-one

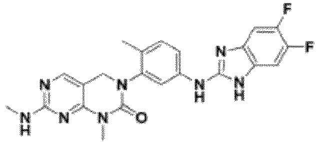

The title compound was obtained by repeating the procedure of Step 3-5 of Example 52, except that 3-(5-amino-2-methylphenyl)-1-methyl-7-(methylamino)-3,4-dihydropyrimido[4,5-d]pyrimidin-2(1H)-one (51.6 mg, 0.173 mmol) was used instead of 3-(5-amino-2-methylphenyl)-1-methyl-7-((6-methylpyridin-3-yl)amino)-3,4-**dihydropyrimido[4,5-d]pyrimidin-2(1H)-one** produced in Step 3-2 of Example 52, and 2-chloro-5,6-difluoro-1H-benzo[d]imidazole (65 mg, 0.346 mmol) was used instead of 2-chloro-5-(trifluoromethyl)-1H-benzo[d]imidazole produced in Step 3-4 of Example 52.

LCMS (ESI+, m/z): 451.20 [M+H]+

### Example 64: 7-(cyclopropylamino)-3-(5-((5,6-difluoro-1H-benzo[d]imidazol-2-yl)amino)-2-methylphenyl)-1-methyl-3,4-dihydropyrimido[4,5-d]pyrimidin-2(1H)-one

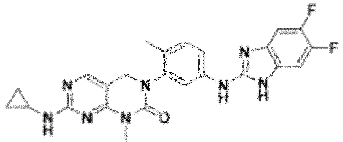

The title compound was obtained by repeating the procedure of Step 3-5 of Example 52, except that 3-(5-amino-2-methylphenyl)-7-(cyclopropylamino)-1-methyl-3,4-dihydropyrimido[4,5-d]pyrimidin-2(1H)-one (56.1 mg, 0.173 mmol) was used instead of 3-(5-amino-2-methylphenyl)-1-methyl-7-((6-methylpyridin-3-yl)amino)-3,4-dihydropyrimido[4,5-d]pyrimidin-2(1H)-one produced in Step 3-2 of Example 52, and 2-chloro-5,6-difluoro-1H-benzo[d]imidazole (65 mg, 0.346 mmol) was used instead of 2-chloro-5-(trifluoromethyl)-1H-benzo[d]imidazole produced in Step 3-4 of Example 52.

LCMS (ESI+, m/z): 477.20 [M+H]+

### [Representative Synthesis Example 4]

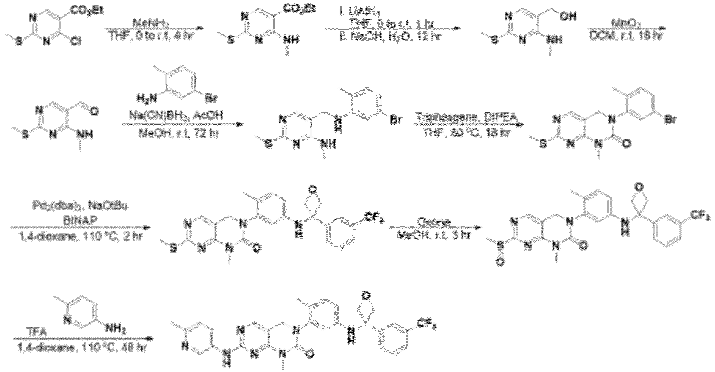

### Example 65: 1-methyl-3-(2-methyl-5-((3-(3-(trifluoromethyl)phenyl)oxetan-3-yl)amino)phenyl)-7-((6-methylpyridin-3-yl)amino)-3,4-dihydropyrimido[4,5-d]pyrimidin-2(1H)-one

### Step 4-1) Ethyl 4-(methylamino)-2-(methylthio)pyrimidine-5-carboxylate

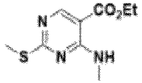

Ethyl 4-chloro-2-(methylthio)pyrimidine-5-carboxylate (5.0 g, 21.6 mmol) was dissolved in anhydrous dry tetrahydrofuran (86.0 mL). After the reaction vessel was cooled to 0°C, 2.0 M methylamine (tetrahydrofuran solution, 32.3 mL, 64.5 mmol) was added dropwise to the reaction vessel, followed by stirring at room temperature for 4 hours. After completion of the reaction, a saturated aqueous solution of sodium hydrogen carbonate was added dropwise to the mixture solution, followed by extraction with ethyl acetate. The combined organic layers were washed with brine, dried with anhydrous sodium sulfate, and then concentrated to obtain 4.4 g (90% yield) of the title compound as a white solid.

LCMS (ESI+, m/z): 228.10 [M+H]+

¹H NMR (400 MHz, CDCl₃) δ 8.61 (s, 1H), 4.31 (q, J = 7.1 Hz, 2H), 3.08 (d, J = 5.0 Hz, 3H), 2.54 (d, J = 1.0 Hz, 3H), 1.45 - 1.20 (m, 3H).

### Step 4-2) (4-(methylamino)-2-(methylthio)pyrimidin-5-yl)methanol

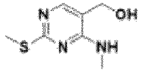

Lithium aluminum hydride (820 mg, 21.0 mmol) was dissolved in anhydrous dry tetrahydrofuran (30.0 mL) and the reaction vessel was cooled to 0°C. Then, ethyl 4-(methylamino)-2-(methylthio)pyrimidine-5-carboxylate (4.0 g, 17.6 mmol) produced in Step 4-1 of Example 65 was dissolved in anhydrous dry tetrahydrofuran (30.0 mL)) and slowly added dropwise to the reaction vessel. The reaction mixture was stirred at room temperature for 1 hour, and water (1.5 mL) was added dropwise thereto, followed by additional stirring for 30 minutes. Subsequently, 10% sodium hydroxide aqueous solution (1.0 mL) was added dropwise thereto, followed by stirring at room temperature for 12 hours. After completion of the reaction, the mixture solution was cooled to room temperature, filtered through a celite pad, and then concentrated using a vacuum distillation device. Water was added to the concentrate, followed by extraction with ethyl acetate. The combined organic layers were dried with anhydrous sodium sulfate and concentrated. The resulting residue was purified by MPLC (ethyl acetate: hexane = 1: 2 (v/v)), and the resulting solution was concentrated under reduced pressure to obtain 2.98 g (92% yield) of the title compound.

LCMS (ESI+, m/z): 186.0 [M+H]+

¹H NMR (400 MHz, DMSO) δ 7.82 (s, 1H), 6.83 (d, J = 4.9 Hz, 1H), 5.07 (s, 1H), 4.29 (d, J = 4.3 Hz, 2H), 2.85 (d, *J* = 4.6 Hz, 3H), 2.42 (s, 3H).

### Step 4-3) (4-(methylamino)-2-(methylthio)pyrimidine-5-carbaldehyde

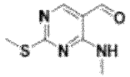

(4-(methylamino)-2-(methylthio)pyrimidin-5-yl)methanol (2.0 g, 10.8 mmol) produced in Step 4-2 of Example 65 was dissolved in dichloromethane (40.0 mL), and manganese dioxide (9.4 g, 108 mmol) was added thereto, followed by stirring at room temperature for 18 hours. After completion of the reaction, the mixture solution was cooled to room temperature. The cooled mixture solution was filtered through a celite pad and concentrated using a vacuum distillation device to obtain 1.78 g (90% yield) of the title compound.

LCMS (ESI+, m/z): 184.10 [M+H]+

¹H NMR (400 MHz, CDCl₃) δ 9.69 (s, 1H), 8.55 (s, 1H), 8.29 (s, 1H), 3.11 (d, *J* = 5.0 Hz, 3H), 2.56 (s, 3H).

### Step 4-4) 5-(((5-bromo-2-methylphenyl)amino)methyl)-N-methyl-2-(methylthio)pyrimidine-4-amine

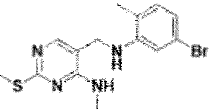

(4-(methylamino)-2-(methylthio)pyrimidine-5-carbaldehyde (800 mg, 4.4 mmol) produced in Step 4-3 of Example 65 was dissolved in methanol (50 mL). Acetic acid (0.5 mL, 8.7 mmol), 5-bromo-2-methylaniline (814 mg, 4.4 mmol) and sodium cyanoborohydride (1.37 g, 21.8 mmol) were added thereto, followed by stirring at room temperature for 72 hours. After completion of the reaction, a saturated aqueous solution of sodium hydrogen carbonate was added dropwise to the mixture solution, followed by extraction with ethyl acetate. The combined organic layers were washed with brine, dried with anhydrous sodium sulfate, and then concentrated. The resulting residue was purified by MPLC (ethyl acetate: hexane = 2: 3 (v/v)), and the resulting solution was concentrated under reduced pressure to obtain 1.23 g (79% yield) of the title compound.

LCMS (ESI+, m/z): 353.10 [M+H]+

¹H NMR (400 MHz, CDCl₃) δ 7.91 (s, 1H), 7.00 - 6.84 (m, 3H), 5.60 (d, *J* = 6.8 Hz, 1H), 4.06 (d, *J* = 5.0 Hz, 2H), 3.34 (d, *J* = 5.5 Hz, 1H), 3.03 (d, *J* = 4.8 Hz, 3H), 2.55 (s, 3H), 2.06 (s, 3H).

### Step 4-5) 3-(5-bromo-2-methylphenyl)-1-methyl-7-(methylthio)-3,4-dihydropyrimido[4,5-d]pyrimidin-2(1H)-one

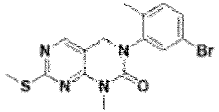

954 mg (89% yield) of the title compound was obtained by repeating the procedure of Step 1-5 of Example 1, except that 5-(((5-bromo-2-methylphenyl)amino)methyl)-N-methyl-2-(methylthio)pyrimidine-4-amine (1.0 g, 2.84 mmol) produced in Step 4-5 of Example 65 was used instead of 5-(((5-(((tert-butyldiphenylsilyl)oxy)methyl)-2-methylphenyl)amino)methyl)-2-chloro-N-methylpyrimidine-4-amine in Step 1-5 of Example 1.

LCMS (ESI+, m/z): 379.0 [M+H]+

¹H NMR (400 MHz, CDCl₃) δ 8.06 (d, *J* = 1.1 Hz, 1H), 7.42 - 7.35 (m, 2H), 7.17 (d, *J* = 8.4 Hz, 1H), 4.70 (dd, *J* = 14.3, 1.1 Hz, 1H), 4.46 (d, *J* = 14.4 Hz, 1H), 3.45 (s, 3H), 2.58 (s, 3H), 2.17 (s, 3H).

### Step 4-6) 2-methyl-N-(3-(3-(trifluoromethyl)phenyl)oxetan-3-yl)propane-2-sulfinamide

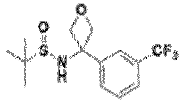

1-bromo-3-(trifluoromethyl)benzene (1.0 g, 4.47 mmol) was dissolved in anhydrous dry tetrahydrofuran (27.0 mL), and the reaction vessel was cooled to -78°C. 1.6 M n-butyllyrium solution (2.73 mL, 4.38 mmol) was slowly added dropwise to the reaction vessel, followed by stirring at - 78°C for 1 hour. Then, oxetane-3-tert-butylsulfinimine (625 mg, 3.57 mmol) was dissolved in anhydrous dry tetrahydrofuran (13.5 mL) and slowly added dropwise to the reaction vessel, followed by stirring at the same temperature for 1 hour. The reaction mixture was warmed to room temperature and a saturated aqueous solution of ammonium chloride was added dropwise thereto, followed by extraction with ethyl acetate. The combined organic layers were washed with brine, dried with anhydrous sodium sulfate, and then concentrated. The resulting residue was purified by MPLC (ethyl acetate: hexane = 1: 3 (v/v)), and the resulting solution was concentrated under reduced pressure to obtain 871 mg (76% yield) of the title compound.

LCMS (ESI+, m/z): 322.10 [M+H]+

¹H NMR (400 MHz, CDCl₃) δ 7.70 - 7.51 (m, 4H), 5.21 (d, *J* = 7.1 Hz, 1H), 5.12 (d, *J* = 7.1 Hz, 1H), 5.04 (d, *J* = 6.9 Hz, 1H), 4.92 (d, *J* = 6.9 Hz, 1H), 4.15 (s, 1H), 1.22 (s, 9H).

### Step 4-7) 3-(3-(trifluoromethyl)phenyl)oxetane-3-amine

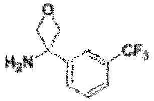

2-methyl-N-(3-(3-(trifluoromethyl)phenyl)oxetan-3-yl)propane-2-sulfinamide (890 mg, 2.77 mmol) produced in Step 4-6 of Example 65 was dissolved in ethyl acetate (5.0 mL). 4 M hydrochloric acid (3.48 mL, 13.9 mmol) was slowly added thereto dropwise, followed by stirring at room temperature for 30 minutes. The white precipitated solid was separated using a filter and washed with ethyl acetate. The resulting material was developed in ethyl acetate (15.0 mL), and a saturated aqueous solution of sodium hydrogen carbonate was added dropwise thereto, followed by extraction with ethyl acetate. The combined organic layers were dried with sodium sulfate and then concentrated to obtain 562 mg (94% yield) of the title compound.

LCMS (ESI+, m/z): 218.10 [M+H]+

¹H NMR (400 MHz, CDCl₃) δ 7.87 - 7.76 (m, 2H), 7.62 - 7.50 (m, 2H), 4.95 (d, J = 6.5 Hz, 2H), 4.77 (d, J = 6.5 Hz, 2H) .

### Step 4-8)1-methyl-3-(2-methyl-5-((3-(3-(trifluoromethyl)phenyl)oxetan-3-yl)amino)phenyl)-7-(methylthio)-3,4-dihydropyrimido[4,5-d]pyrimidin-2(1H)-one

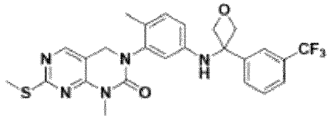

3-(5-bromo-2-methylphenyl)-1-methyl-7-(methylthio)-3,4-dihydropyrimido[4,5-d]pyrimidin-2(1H)-one (200 mg, 0.529 mmol), produced in Step 4-5 of Example 65, and 3-(3-(trifluoromethyl)phenyl)oxetane-3-amine (170 mg, 0.783 mmol) produced in Step 4-7 of Example 65 were dissolved in 1,4-dioxane (1.0 mL). Pd₂(dba)₃ (96 mg, 0.105 mmol), sodium tert-butoxide (102 mg, 1.06 mmol) and BINAP (65 mg, 0.105 mmol) were added thereto, followed by stirring under reflux at 110°C for 2 hours. After completion of the reaction, the mixture solution was cooled to room temperature, filtered through a celite pad, and concentrated using a vacuum distillation device. The resulting residue was purified by MPLC (ethyl acetate: dichloromethane = 2: 1 (v/v)), and the resulting solution was concentrated under reduced pressure to obtain 98 mg (36% yield) of the title compound.

LCMS (ESI+, m/z): 516.20 [M+H]+

¹H NMR (400 MHz, CDCl₃) δ 7.98 (d, *J* = 20.7 Hz, 2H), 7.86 (d, *J* = 7.9 Hz, 1H), 7.58 (d, *J* = 7.7 Hz, 1H), 7.52 (d, *J* = 7.8 Hz, 1H), 6.97 (d, *J* = 8.3 Hz, 1H), 6.28 (d, *J* = 2.4 Hz, 1H), 6.01 (dd, *J* = 8.3, 2.5 Hz, 1H), 5.02 - 4.83 (m, 4H), 4.55 - 4.46 (m, 1H), 4.41 (d, *J* = 14.5 Hz, 1H), 3.43 (s, 3H), 2.57 (s, 3H), 2.06 (s, 3H).

### Step 4-9) 1-methyl-3- (2-methyl-5-((3-(3-(trifluoromethyl)phenyl)oxetan-3-yl)amino)phenyl)-7-(methylsulfinyl)-3,4-dihydropyrimido[4,5-d]pyrimidin-2(1H)-one

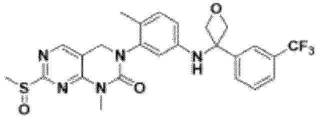

**1-methyl-3-(2-methyl-5-((3-(3-**(trifluoromethyl)phenyl)oxetan-3-yl)amino)phenyl)-7-(methylthio)-3,4-dihydropyrimido[4,5-d]pyrimidin-2(1H)-one (26 mg, 0.051 mmol) produced in Step 4-8 of Example 65 was dissolved in methanol (0.5 mL), and potassium peroxymonosulfate (74 mg, 0.24 mmol) was added thereto, followed by stirring at room temperature for 3 hours. After completion of the reaction, water was added to the mixture solution, followed by extraction with dichloromethane and then concentration using a vacuum distillation device to obtain 17 mg (63% yield) of the title compound.

LCMS (ESI+, m/z): 532.20 [M+H]+

### Step 4-10) 1-methyl-3-(2-methyl-5-((3-(3-(trifluoromethyl)phenyl)oxetan-3-yl)amino)phenyl)-7-((6-methylpyridin-3-yl)amino)-3,4-dihydropyrimido[4,5-d]pyrimidin-2(1H)-one

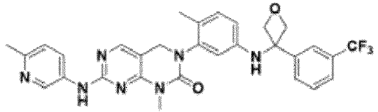

1-methyl-3-(2-methyl-5-((3-(3-(trifluoromethyl)phenyl)oxetan-3-yl)amino)phenyl)-7-(methylsulfinyl)-3,4-dihydropyrimido[4,5-d]pyrimidin-2(1H)-one (10.0 mg, 0.018 mmol), produced in Step 4-9 of Example 65, and 6-methylpyridine-3-amine (3.9 mg, 0.036 mmol) were dissolved in 1,4-dioxane (0.4 mL). Trifluoroacetic acid (2.75 µL, 0.036 mmol) was added thereto, followed by stirring under reflux at 110°C for 48 hours. The mixture solution was cooled to room temperature and then concentrated using a vacuum distillation device. The resulting residue was purified by HPLC (high-performance liquid chromatography/acetonitrile: water = 5: 95 → 95: 5 (v/v), 40 minutes), and the resulting solution was freezedried to obtain 2.8 mg (27% yield) of the title compound as a white solid.

LCMS (ESI+, m/z): 576.25 [M+H]+

¹H NMR (400 MHz, MeOD) δ 9.49 (d, *J* = 2.5 Hz, 1H), 8.46 (dd, *J* = 8.8, 2.6 Hz, 1H), 8.16 (s, 1H), 8.01 - 7.96 (m, 1H), 7.95 (s, 1H), 7.81 (d, *J* = 8.9 Hz, 1H), 7.66 - 7.55 (m, 2H), 7.00 (d, *J* = 8.4 Hz, 1H), 6.28 (d, *J* = 2.4 Hz, 1H), 6.15 (dd, *J* = 8.3, 2.5 Hz, 1H), 5.03 - 4.94 (m, 4H), 4.59 (d, *J* = 14.4 Hz, 1H), 4.49 (d, *J* = 14.5 Hz, 1H), 3.43 (s, 3H), 2.72 (s, 3H), 2.05 (s, 3H).

### [Experimental Examples]

### Experimental Example 1. Inhibitory Activities against GCK and ACK1 Kinases

The inhibitory activities of the compounds of the present disclosure against two kinases, GCK and ACK1, were measured, and the IC₅₀ values thereof were calculated. The calculated IC₅₀ values are shown in Table 1 below.

**[Table 1]**

| Test compound | Kinase inhibitory activity, IC₅₀ | |
|---|---|---|
| | ACK1 | GCK |
| Compound No. 1 | A | B |
| Compound No. 3 | A | B |
| Compound No. 4 | A | B |
| Compound No. 5 | A | B |
| Compound No. 6 | A | C |
| Compound No. 7 | A | B |
| Compound No. 9 | B | D |
| Compound No. 10 | A | B |
| Compound No. 11 | C | D |
| Compound No. 12 | B | D |
| Compound No. 13 | C | D |
| Compound No. 14 | B | B |
| Compound No. 18 | A | C |
| [Classification of IC₅₀] A: less than 0.1 µM, B: 0.1 to 1.0 µM, C: 1.0 µM to 10.0 µM, D: more than 10 µM | | |

### Experimental Example 2. Proliferation Inhibitory Activity

The inhibitory activities of the compounds of the present disclosure against the proliferation of the mt-NRAS (G12D) Ba/F3 and OCI-AML3 (mt-NRAS) cell lines were measured, and the GI₅₀ values thereof were calculated. The calculated GI₅₀ values are shown in Table 2 below.

**[Table 2]**

| Test compound | Proliferation inhibitory activity (GI₅₀, µM) | |
|---|---|---|
| | OCI-AML3 (N-Ras Q61L) | Ba/F3 (N-Ras G12D) |
| Compound No. 1 | A | A |
| Compound No. 2 | B | B |
| Compound No. 3 | A | A |
| Compound No. 4 | A | A |
| Compound No. 5 | A | A |
| Compound No. 6 | A | A |
| Compound No. 7 | A | A |
| Compound No. 8 | C | B |
| Compound No. 9 | B | A |
| Compound No. 10 | A | A |
| Compound No. 11 | B | B |
| Compound No. 12 | A | B |
| Compound No. 13 | A | C |
| Compound No. 14 | B | B |
| Compound No. 15 | D | C |
| Compound No. 16 | D | D |
| Compound No. 17 | D | D |
| Compound No. 18 | D | B |
| [Classification of GI₅₀] A: less than 0.5 µM, B: 0.5 to 3.0 µM, C: 3.0 µM to 5.0 µM, D: more than 5.0 µM | | |

Referring to the results in Table 2, it can be seen that the compound of the present disclosure has inhibitory activity against the proliferation of the human acute myeloid leukemia cell line (OCI-AML3) having the NRAS mutant gene, and the effect thereof is remarkable. Therefore, it can be seen that the compound of the present disclosure is particularly effective as a therapeutic agent for acute myeloid leukemia (AML).

### [Formulation Examples]

Meanwhile, the novel compound represented by Formula 1 according to the present disclosure may be formulated in various forms depending on the intended use thereof. The following exemplifies several formulation methods containing, as an active ingredient, the compound represented by Formula 1 according to the present disclosure, but the scope of the present disclosure is not limited thereto.

### Formulation Example 1: Tablet (Direct Compression)

5.0 mg of the active ingredient was sieved and then mixed with 14.1 mg of lactose, 0.8 mg of crospovidone USNF and 0.1 mg of magnesium stearate, and the mixture was compressed into a tablet.

### Formulation Example 2: Tablet (Wet Granulation)

5.0 mg of the active ingredient was sieved and then mixed with 16.0 mg of lactose and 4.0 mg of starch. 0.3 mg of polysorbate 80 was dissolved in pure water, and then a suitable amount of the solution was added to the mixture, followed by atomization to obtain fine particles. After drying, the fine particles were sieved and then mixed with 2.7 mg of colloidal silicon dioxide and 2.0 mg of magnesium stearate. The fine particles were compressed into a tablet.

### Formulation Example 3: Powder and Capsule

5.0 mg of the active ingredient was sieved and then mixed with 14.8 mg of lactose, 10.0 g of polyvinylpyrrolidone and 0.2 mg of magnesium stearate. A No. 5 hard gelatin capsule using a suitable device was filled with the mixture.

### Formulation Example 4: Formulation Injection

A formulation for injection containing 100 mg of the active ingredient, 180 mg of mannitol, 26 mg of Na₂HPO₄·12H₂O and 2,974 mg of distilled water was prepared.

Although the embodiments of the present disclosure have been described above, those skilled in the art to which the present disclosure pertains will understand that the present disclosure may be embodied in other specific forms without departing from the technical spirit or essential features thereof. Therefore, it should be understood that the embodiments described above are illustrative in all respects, not restrictive.

## Claims

1. A compound selected from among a 7-amino-3,4-dihydropyrimidopyrimidin-2-one derivative compound represented by the following Formula 1, a pharmaceutically acceptable salt thereof, a hydrate thereof, and a stereoisomer thereof: 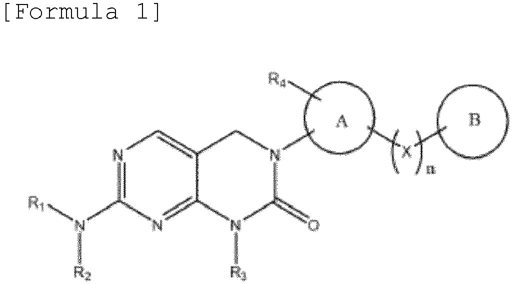 wherein
R₁ is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C (O)- (C₁-C₁₃ alkyl), or R₁ together with a nitrogen atom to which R₂ is attached forms a 4- to 7-membered saturated, unsaturated or aromatic ring, which may optionally contain at least one of N, O, S, NH, C=N, C=O, -NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ is hydrogen, a C₁-C₁₃ alkyl group, a C₃-C₁₀ cyclyl group, or a C₃-C₁₀ heterocyclyl group, or R₂ together with a nitrogen atom to which R₁ is attached forms a 4- to 7-membered saturated, unsaturated or aromatic ring, which may optionally contain at least one of N, O, S, NH, C=N, C=O, - NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₃ is hydrogen, a C₁-C₁₃ alkyl group, a C₃-C₁₀ cyclyl group, or a C₃-C₁₀ heterocyclyl group;
R₄ is hydrogen, a hydroxyl group, a halogen group, a C₁-C₁₃ alkyl group, a C₁-C₆ alkoxy group, a C₁-C₆ alkenyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl) ;
A is a C₆-C₁₀ aryl group, a C₃-C₁₀ cyclyl group, a C₃-C₁₀ heteroaryl group, or a C₃-C₁₀ heterocyclyl group;
n is an integer ranging from 0 to 3;
when n is more than 1, X is each independently selected from the group consisting of -CH₂-, -CR₅R₆-, -O-, - OC(O)-, -C(O)O-, -OS(O)₂-, -S(O)₂O-, -NR₆-, -NR₆CH₂-, - NR₆C(O)-, -C(O)NR₆-, -NR₆C(O)NR₆-, -S(O)₂-, -NR₆S(O)₂-, and - S(O)₂NR₆-;
B is a C₆-C₁₀ aryl group, a C₃-C₁₀ cyclyl group, a C₃-C₁₀ heteroaryl group, or C₃-C₁₀ heterocyclyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ are each independently hydrogen, a C₁-C₆ alkyl group, a C₁-C₆ alkenyl group, a C₁-C₆ alkynyl group, a C₆-C₁₀ aryl group, a C₃-C₁₀ heteroaryl group, or a C₃-C₁₀ heterocyclyl group, or R₅ together with a nitrogen or carbon atom to which R₆ is attached forms a 3- to 7-membered saturated ring, which may optionally contain at least one of N, O, S, NH, C=N, C=O, -NHC(O)-, -NHC(O)NH-, - NHS(O)₂- and SO₂ and may optionally be substituted with at least one of hydrogen, a C₁-C₁₃ alkyl group, a C₆-C₁₀ aryl group, a C₃-C₁₀ heteroaryl group, a hydroxyl group, a halide group and a cyano group; and
the C₃-C₁₀ heteroaryl group and the C₃-C₁₀ heterocyclyl group contain at least one heteroatom selected from the group consisting of N, O and S.

2. The compound of claim 1, wherein B is any one selected from the group consisting of a C₁-C₆ alkyl group, benzene, thiazole, thiophene, pyrazole, benzothiophene, pyridazine, pyrazine, imidazole, oxadiazole, triazole, furan, pyrimidine, oxazole, pyrrole, pyridine, oxadiazole, triazine, thiadiazole, isoxazole, and tetrazole, and B is substituted or unsubstituted.

3. The compound of claim 1, wherein R₂ is hydrogen, R₃ is methyl, and R₄ is methyl.

4. The compound of claim 1, wherein R₁ is any one selected from the group consisting of hydrogen, a C₁-C₆ alkyl group, a C₃-C₁₀ cyclyl group, benzene, thiazole, thiophene, pyrazole, benzothiophene, pyridazine, pyrazine, imidazole, oxadiazole, triazole, furan, pyrimidine, oxazole, pyrrole, pyridine, oxadiazole, triazine, thiadiazole, isoxazole, and tetrazole, and R₁ is substituted or unsubstituted.

5. The compound of claim 1, wherein the compound is any one selected from the group consisting of the following Compound Nos. 1 to 65:
(Compound No. 1)
3-(5-(5-(2-methoxyphenyl)-1H-imidazol-2-yl)-2-methylphenyl)-1-methyl-7-((1-methyl-1H-pyrazol-4-yl)amino)-3,4-dihydropyrimido[4,5-d]pyrimidin-2(1H)-one;
(Compound No. 2)
7-((2,6-dimethylpyridin-3-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 3)
3-(5-(5-(2-methoxyphenyl)-1H-imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 4)
7-((1-(1-ethylpiperidin-4-yl)-1H-pyrazol-4-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 5)
7-((6-(1-ethylpiperidin-4-yl)pyridin-3-yl)amino)-3-(5-(5-(2-methoxyphenyl)-1H-imidazole-2-yl)-2-methylphenyl)-1-methyl-3,4-dihydropyrimido[4,5-d]pyrimidin-2(1H)-one;
(Compound No. 6)
7-((6-(4-acetylpiperazin-1-yl)pyridin-3-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 7)
7-((1-(1-acetylpiperidin-4-yl)-1H-pyrazol-4-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 8)
7-((2,6-dimethylpyridin-3-yl)amino)-1-methyl-3-(2-methyl-5-(5-(2-(trifluoromethoxy)phenyl)-1H-imidazole-2-yl)phenyl)-3,4-dihydropyrimido[4,5-d]pyrimidin-2(1H)-one;
(Compound No. 9)
1-methyl-3-(2-methyl-5-(5-(2-(trifluoromethoxy)phenyl)-1H-imidazol-2-yl)phenyl)-7-((6-methylpyridin-3-yl)amino)-3,4-dihydropyrimido[4,5-d]pyrimidin-2(1H)-one;
(Compound No. 10)
7-((1-(1-ethylpiperidin-4-yl)-1H-pyrazol-4-yl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 11)
7-(cyclopropylamine)-3-(5-(5-(2,3-difluorophenyl)-1H-imidazol-2-yl)-2-methylphenyl)-1-methyl-3,4-dihydropyrimido[4,5-d]pyrimidin-2(1H)-one;
(Compound No. 12)
3-(5-(5-(2,3-difluorophenyl)-1H-imidazol-2-yl)-2-methylphenyl)-1-methyl-7-((6-methylpyridin-3-yl))amino)-3,4-dihydropyrimido[4,5-d]pyrimidin-2(1H)-one;
(Compound No. 13)
1-methyl-3-(2-methyl-5-(5-(3-(trifluoromethyl)phenyl)-1H-imidazol-2-yl)phenyl)-7-((6-methylpyridin-3-yl)amino)-3,4-dihydropyrimido[4,5-d]pyrimidin-2(1H)-one;
(Compound No. 14)
7-((6-(4-acetylpiperazin-1-yl)pyridin-3-yl)amino)-1-methyl-3-(2-methyl-5-(5-(2-(trifloromethoxy)phenyl)-1H-imidazol-2-yl)phenyl)-3,4-dihydropyrimido[4,5-d]pyrimidin-2(1H)-one;
(Compound No. 15)
7-((6-(1-ethylpiperidin-4-yl)pyridin-3-yl)amino)-1-methyl-3-(2-methyl-5-(5-(2-(trifloromethoxy)phenyl)-1H-imidazol-2-yl)phenyl)-3,4-dihydropyrimido[4,5-d]pyrimidin-2(1H)-one;
(Compound No. 16)
7-((6-(4-ethylpiperazin-1-yl)pyridin-3-yl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 17)
7-((1-(1-acetylpiperidin-4-yl)-1H-pyrazol-4-yl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 18)
1-methyl-3-(2-methyl-5-(5-(2-(trifluoromethoxy)phenyl)-1H-imidazol-2-yl)phenyl)-7-( (4-morpholinophenyl)amino)-3,4-dihydropyrimido[4,5-d]pyrimidin-2(1H)-one;
(Compound No. 19)
1-methyl-3-(2-methyl-5-(5-(2-(trifluoromethoxy)phenyl)-1H-imidazol-2-yl)phenyl)-7-(phenylamino)-3,4-dihydropyrimido[4,5-d]pyrimidin-2(1H)-one;
(Compound No. 20)
7-((4-methoxybenzyl)amino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 21)
7-(benzylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 22)
7-(cyclopropylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 23)
7-(cyclopentylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 24)
7-(cyclohexylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 25)
7-(butylamino)-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 26)
1-methyl-3-(2-methyl-5-(5-(2-(trifluoromethoxy)phenyl)-1H-imidazol-2-yl)phenyl)-7-(methylamino)-3,4-dihydropyrimido[4,5-d]pyrimidin-2(1H)-one;
(Compound No. 27)
7-amino-1-methyl-3-(2-methyl-5-(5-(2-(trifluoromethoxy)phenyl)-1H-imidazol-2-yl)phenyl)-3,4-dihydropyrimido[4,5-d]pyrimidin-2(1H)-one;
(Compound No. 28)
7-((6-(4-ethylpiperazin-1-yl)pyridin-3-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 29)
7-((1-(1-acetylpiperidin-4-yl)-1H-pyrazol-4-yl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 30)
3-(5-(5-(2-methoxyphenyl)-1H-imidazol-2-yl)-2-methylphenyl)-1-methyl-7-(phenylamino)-3,4-dihydropyrimido[4,5-d]pyrimidin-2(1H)-one;
(Compound No. 31)
7-(benzylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 32)
7-((4-methoxybenzyl)amino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 33)
3-(5-(5-(2-methoxyphenyl)-1H-imidazol-2-yl)-2-methylphenyl)-1-methyl-7-(methylamino)-3,4-dihydropyrimido[4,5-d]pyrimidin-2(1H)-one;
(Compound No. 34)
7-(cyclopropylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 35)
7-(butylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 36)
7-(cyclopentylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 37)
7-(cyclohexylamino)-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 38)
7-amino-3-(5-(5-(2-methoxyphenyl)-1H-imidazol-2-yl)-2-methylphenyl)-1-methyl-3,4-dihydropyrimido[4,5-d]pyrimidin-2(1H)-one;
(Compound No. 39)
1-methyl-3-(2-methy1-5-(6-(trifluoromethyl)-1H-benzo[d]imidazol-2-yl)phenyl)-7-((6-methylpyridin-3-yl)amino)-3,4-dihydropyrimido[4,5-d]pyrimidin-2(1H)-one;
(Compound No. 40)
3-(5-(5,6-difluoro-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 41)
3-(5-(5,6-dibromo-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 42)
1-methyl-3-(2-methyl-5-(7-methyl-1H-benzo[d]imidazol-2-yl)phenyl)-7-((6-methylpyridin-3-yl)amino)-3,4-dihydropyrimido[4,5-d]pyrimidin-2(1H)-one;
(Compound No. 43)
3-(5-(6-bromo-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 44)
3-(5-(6-chloro-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 45)
1-methyl-3-(2-methyl-5-(6-methyl-1H-benzo[d]imidazol-2-yl)phenyl)-7-((6-methylpyridin-3-yl)amino)-3,4-dihydropyrimido[4,5-d]pyrimidin-2(1H)-one;
(Compound No. 46)
3-(5-(5,6-dichloro-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 47)
3-(5-(5,6-dimethyl-1H-benzo[d]imidazol-2-yl)-2-methylphenyl)-1-methyl-7-((6-methylpyridin-3-yl)amino)-3,4-dihydropyrimido[4,5-d]pyrimidin-2(1H)-one;
(Compound No. 48)
1-methyl-3-(2-methyl-5-(6-(trifluoromethyl)-1H-benzo[d]imidazol-2-yl)phenyl)-7-(phenylamino)-3,4-dihydropyrimido[4,5-d]pyrimidin-2(1H)-one;
(Compound No. 49)
1-methyl-3-(2-methyl-5-(6-(trifluoromethyl)-1H-benzo[d]imidazol-2-yl)phenyl)-7-(phenylamino)-3,4-dihydropyrimido[4,5-d]pyrimidin-2(1H)-one;
(Compound No. 50)
7-(cyclopropylamino)-1-methyl-3-(2-methyl-5-(6-(trifluoromethyl)-1H-benzo[d]imidazol-2-yl)phenyl)-3,4-dihydropyrimido[4,5-d]pyrimidin-2(1H)-one;
(Compound No. 51)
7-((2,6-dimethylpyridin-3-yl)amino)-1-methyl-3-(2-methyl-5-(6-(trifluoromethyl)-1H-benzo[d]imidazol-2-yl)phenyl)-3,4-dihydropyrimido[4,5-d]pyrimidin-2(1H)-one;
(Compound No. 52)
1-methyl-3-(2-methyl-5-(6-(trifluoromethyl)-1H-benzo[d]imidazol-2-yl)phenyl)-7-((6-methylpyridin-3-yl)amino)-3,4-dihydropyrimido[4,5-d]pyrimidin-2(1H)-one;
(Compound No. 53)
3-(5-((5,6-difluoro-1H-benzo[d] imidazol-2-yl) amino)-2-methylphenyl)-1-methyl-7-((6-methylpyridin-3-yl)amino)-3,4-dihydropyrimido[4,5-d]pyrimidin-2(1H)-one;
(Compound No. 54)
3-(5-((1H-benzo[d]imidazol-2-yl)amino)-2-methylphenyl)-1-methyl-7-((6-methylpyridin-3-yl)amino)-3,4-dihydropyrimido[4,5-d]pyrimidin-2(1H)-one;
(Compound No. 55)
3-(5-((6-bromo-1H-benzo[d]imidazol-2-yl) amino)-2-methylphenyl)-1-methyl-7-((6-methylpyridin-3-yl)amino)-3,4-dihydropyrimido[4,5-d]pyrimidin-2(1H)-one;
(Compound No. 56)
3-(5-((6-chloro-1H-benzo[d]imidazol-2-yl) amino)-2-methylphenyl)-1-methyl-7-((6-methylpyridin-3-yl)amino)-3,4-dihydropyrimido[4,5-d]pyrimidin-2(1H)-one;
(Compound No. 57)
1-methyl-3-(2-methyl-5-((6-methyl-1H-benzo[d]imidazol-2-yl)amino)phenyl)-7-((6-methylpyridin-3-yl)amino)-3,4-dihydropyrimido[4,5-d]pyrimidin-2(1H)-one;
(Compound No. 58)
3-(5-((6-dibromo-1H-benzo[d]imidazol-2-yl) amino)-2-methylphenyl)-1-methyl-7-((6-methylpyridin-3-yl)amino)-3,4-dihydropyrimido[4,5-d]pyrimidin-2(1H)-one;
(Compound No. 59)
3-(5-((6-dichloro-1H-benzo[d]imidazol-2-yl) amino)-2-methylphenyl)-1-methyl-7-((6-methylpyridin-3-yl)amino)-3,4-dihydropyrimido[4,5-d]pyrimidin-2(1H)-one;
(Compound No. 60)
3-(5-((5,6-dimethyl-1H-benzo[d]imidazol-2-yl) amino)-2-methylphenyl)-1-methyl-7-((6-methylpyridin-3-yl)amino)-3,4-dihydropyrimido[4,5-d]pyrimidin-2(1H)-one;
(Compound No. 61)
3-(5-((5,6-difluoro-1H-benzo[d] imidazol-2-yl) amino)-2-methylphenyl)-1-methyl-7-(phenylamino)-3,4-dihydropyrimido[4,5-d]pyrimidin-2(1H)-one;
(Compound No. 62)
3-(5-((5,6-difluoro-1H-benzo[d] imidazol-2-yl) amino)-2-methylphenyl)-7-((2,6-dimethylpyridin-3-yl)amino)-1-methyl-3,4-dihydropyrimido[4,5-d]pyrimidin-2(1H)-one;
(Compound No. 63)
3-(5-((5,6-difluoro-1H-benzo[d] imidazol-2-yl) amino)-2-methylphenyl)-1-methyl-7-(methylamino)-3,4-dihydropyrimido[4,5-d]pyrimidin-2(1H)-one;
(Compound No. 64)
7-(cyclopropylamino)-3-(5-((5,6-difluoro-1H-benzo[d]imidazol-2-yl) amino)-2-methylphenyl)-1-methyl-3,4-dihydropyrimido[4,5-d]pyrimidin-2(1H)-one; and
(Compound No. 65)
1-methyl-3-(2-methyl-5-((3-(3-(trifluoromethyl)phenyl)oxetan-3-yl)amino)phenyl)-7-((6-methylpyridin-3-yl)amino)-3,4-dihydropyrimido[4,5-d]pyrimidin-2(1H)-one.

6. The compound of claim 1, wherein the pharmaceutically acceptable salt is a salt of an inorganic acid or organic acid selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

7. A pharmaceutical composition for preventing, alleviating or treating cancer containing the compound of any one of claims 1 to 6 as an active ingredient.

8. The pharmaceutical composition of claim 7, wherein the cancer is caused by NRAS mutation.

9. The pharmaceutical composition of claim 7, which is applied to a patient with NRAS mutation.

10. The pharmaceutical composition of claim 7, wherein the cancer is at least one selected from the group consisting of melanoma, colorectal cancer, lung cancer, bladder cancer, thyroid cancer, multiple myeloma, and blood cancer.

11. The pharmaceutical composition of claim 7, wherein the cancer is acute myeloid leukemia (AML).

12. The pharmaceutical composition of claim 7, which is administered to a patient with NRAS G12D.
